# EUROPEAN PATENT APPLICATION

(11) **EP 3 459 964 A1**
(43) Date of publication of application: **27.03.2019**
(21) Application number: 17193148.8
(22) Date of filing: 26.09.2017
(51) Int. Cl.: C07K 7/08, A61K 49/14

(54) **GALECTIN-1 BINDING PEPTIDES AND USE THEREOF FOR DIAGNOSTIC AND THERAPEUTIC PURPOSE**

(71) Applicant: Université de Mons, 7000 Mons (BE)
(72) Inventor: Burtea, Carmen, 7000 Mons (BE); Laurent, Sophie, 7300 Boussu (BE); Muller, Robert, 7000 Mons (BE); Stanicki, Dimitri, 6141 Forchies-la-Marche (BE); Fanfone, Déborah, 7301 Hornu (BE); Saussez, Sven, 1180 Bruxelles (BE)
(74) Representative: Brantsandpatents bvba

(57) **Abstract**

The current invention concerns galectin-1 binding peptide, preferably linked to a tracer or contrast agent suitable for being used in a medical imaging methodology and therapeutic use.

## Description

### Technical field

The invention pertains to the technical field of peptides binding to galectin-1 and their use in medical imaging, particularly for aid in the imaging malignancies of the thyroid gland and diagnosis of thyroid cancer or malignancies of the thyroid gland.

### Background

During these last decades, worldwide incidence of thyroid cancer has increased. According to the American Cancer Society, about 64,300 new cases of thyroid cancer were diagnosed in 2016, with a clear predominance in women (48,167 in women vs. 16,133 in men) and about 1,980 deaths caused by this pathology (1,131 women vs. 849 men) (National Cancer Institute, 2016). In addition to the exposition to ionizing radiation, this relatively high incidence of thyroid cancers can be explained by the overdiagnosis of nodules and by the better detection of small papillary carcinomas (<1 cm in size). These nodules are commonly found in general population (19 to 67%), their prevalence being from 4 to 7 %. Nodule suspicion after palpation is often confirmed with ultrasound and the fine-needle aspiration cytology (FNAC). Despite its sensitivity, FNAC does not allow to distinguish between benign and malign diseases for 15 to 30% of the cases hence increasing the rate of false positives. Due to increased imaging studies performed on patients for unrelated pathology, these nodules are also found by chance.

The treatment administrated to these patients is radioactive iodine after partial/total thyroidectomy. However, 80 to 85% of these thyroid proliferations are benign. Only 1 to 5% of thyroid nodules are malignant and most of them (60-70% of thyroid cancers) are micropapillary thyroid cancers with excellent long-term prognosis. These surgeries are painful for the patients and imply a cost for society. Due to this challenge, there is a need in the art for method for visualizing malignancies of the thyroid or thyroid nodules in a non-invasive manner, and for providing tools to diagnose well-differentiated thyroid cancers.

Galectin-1 (gal-1) is a protein known to be overexpressed in thyroid cancer. In cancer, gal-1 is secreted and contributes to adhesion to the extracellular matrix, opposed to a healthy thyroid or nodules where the gal-1 expression is predominantly intracellular. High expression of gal-1 is linked to poor prognostic parameters in human cancers (tumor aggressiveness, metastasis, advanced cancer stage, poor survival, cytotoxic drug resistance). Gal-1 may thus be considered as an interesting biomarker for thyroid tumors.

Gal-1 has already been targeted in order to inhibit its pro-tumoral activities by several kinds of compounds varying from antibodies to oligosaccharide derivatives. Most of these antagonists are glycomimetics and β-galactoside analogues targeting the carbohydrate recognition domain (CRD). Despite their effects on tumor development, these compounds have some limitations in terms of pharmacokinetics and pharmacodynamics.

Although some progress has been made, there remains a need in the art for a visualization method which allows distinguishing between malign and benign thyroid nodules and notably the papillary thyroid cancer.

The invention thereto aims to provide a solution for the latter problem.

### Summary of the invention

The present invention provides peptides according to claim 1, which are able to bind to gal-1 *in vivo.* By linking these peptides to a tracer or contrast agent, the peptides may be used for *in vivo* medical imaging in a non-invasive manner. Hence, a method for imaging gal-1 expressing tissues and/or organs in a subject according to claim 9 is disclosed.

Since malignancies or cancer of the thyroid has been reported to be linked to high levels of gal-1, the current invention also pertains to a diagnostic composition according to claim 8 and a method for providing information regarding thyroid cancer diagnosis or malignancies according to claim 14.

The current invention provides a non-invasive manner to visualize gal-1 positive tissues or organs and provides a particularly useful diagnostic tool for evaluating whether or not a malignancy is present in the thyroid gland and if indeed the case, helps to evaluate the type of thyroid cancer.

Secondly, the current invention provides tools which can be used for therapeutic treatment of malignancies in thyroid glands and/or nodules.

### Description of figures

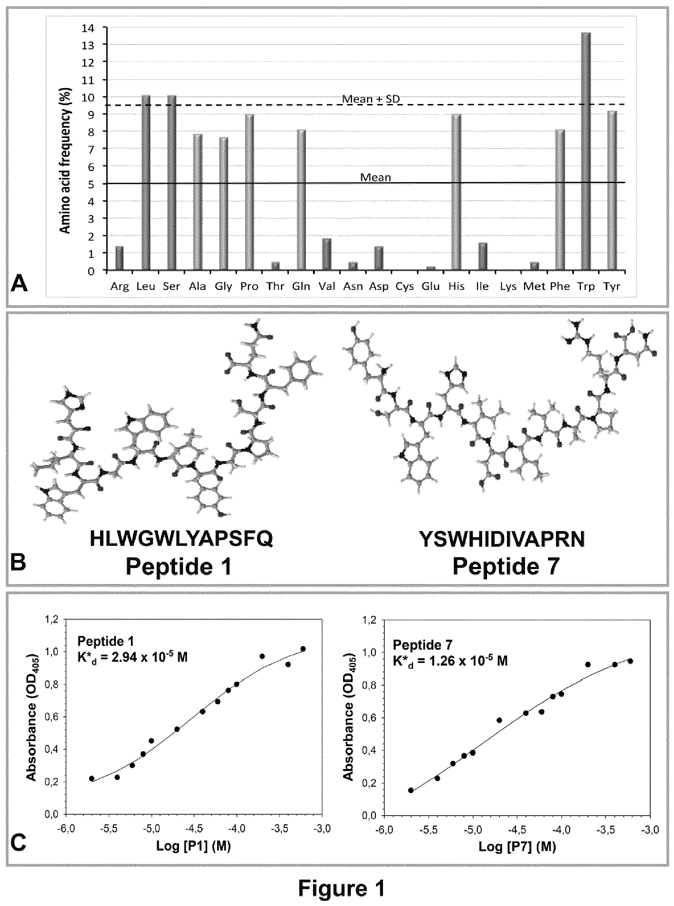

**Figure 1** shows the amino acid composition of the peptides selected after the two phage display experiments and the apparent dissociation constant (K*_{d}) values of the selected peptides: (A) amino acid frequency in the structure of 8 identified peptides; (B) three-dimensional structures of P1 and P7 (Chemsketch 12.01, 2010); (C) the titration curves and K*_{d} values against galectin-1 of P1 and P7.

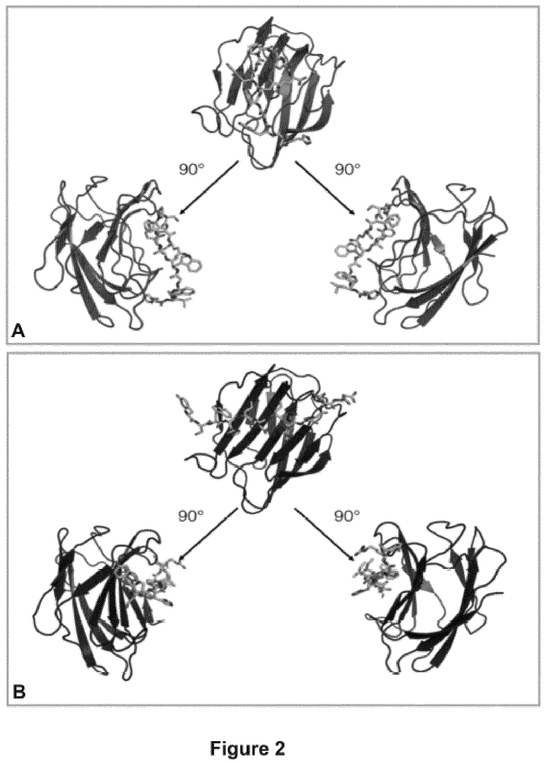

**Figure 2** shows a docking simulation of peptide 1 (A) and 7 (B) with gal-1 performed by CABS dock method.

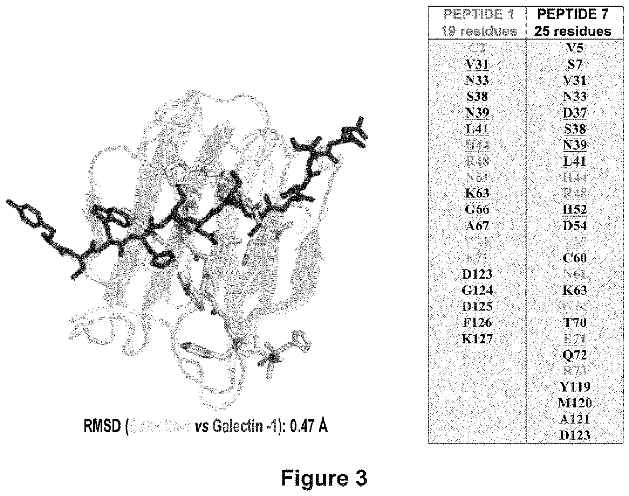

**Figure 3** shows a comparison of gal-1 structures when interacting with peptide 1 (in light grey) or 7 (in black). On the left: Stick representation (heavy atoms) of peptides, the gal-1 structure being depicted shaded. On the right: list of residues of gal-1 located at max. 4 Å from the peptides.

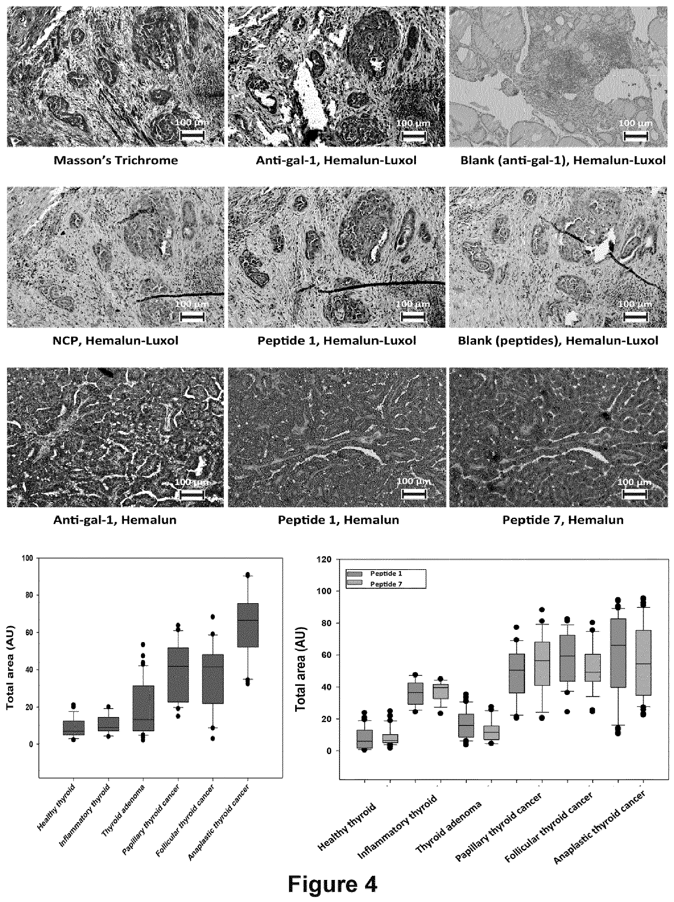

**Figure 4** shows an immunostaining of gal-1 expression in papillary thyroid cancer biopsies using biotinylated P1 and P7 or anti-gal-1 antibody. Blanks correspond to the negative controls (primary antibody excluded), whereas NCP is a negative control peptide. The box-and-whisker plots show the expression of gal-1 (left) and the binding of peptides P1 and P7 (right) in healthy thyroid and in various pathological conditions..

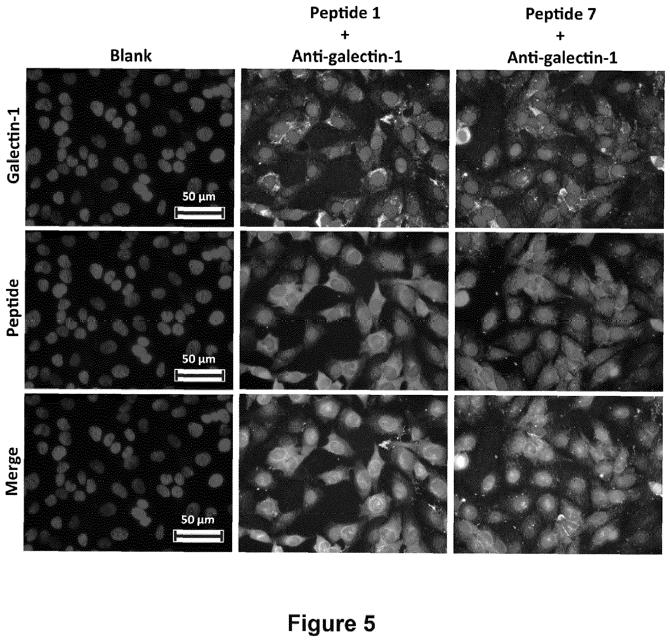

**Figure 5** shows a co-localization of P1 and P7 with gal-1 expressed by human thyroid papillary carcinoma-1 (TPC-1) cells by immunofluorescence. Gal-1 is stained in green by Dylight 488 (first horizontal line, here shown in grayscale), peptides were stained in red by Dylight 594 (second horizontal line, here shown in grayscale), whereas nuclei are stained in blue with DAPI (shown in grayscale). The co-localization is observed by the yellow-orange color of the merged microphotographs (third line, merge, shown in greyscale).

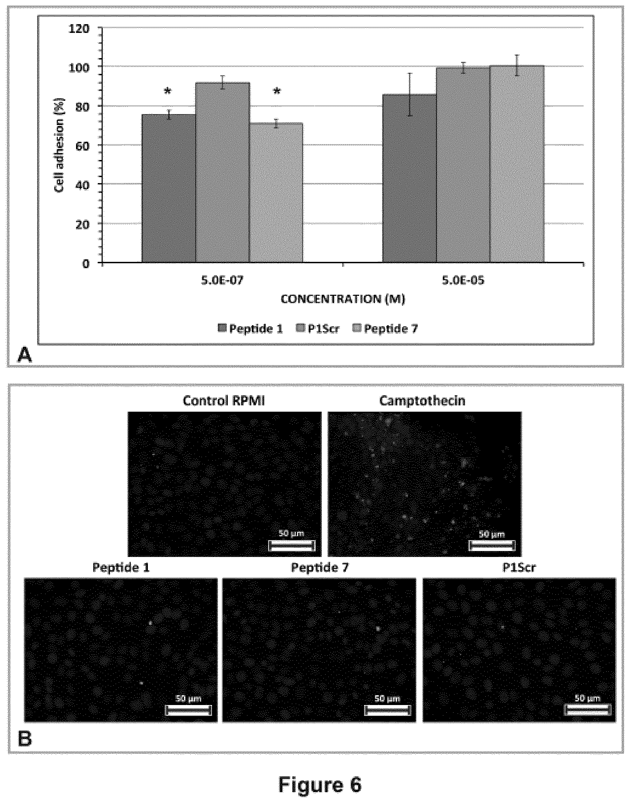

**Figure 6** shows the effect of peptides (P1, P7 and P1Scr) on the adhesion (A) and cell death by apoptosis (**B**) of TPC-1 cells. Apoptosis was detected with an anti-active caspase-3 antibody stained in green with Dylight 488 (shown in grayscale). Nuclei are stained in blue with DAPI (shown in grayscale). Negative control cells were incubated with medium alone (control RPMI), whereas positive control cells were treated with camptothecin to induce apoptosis. * = p<0.05 as compared to non-inhibited cells.

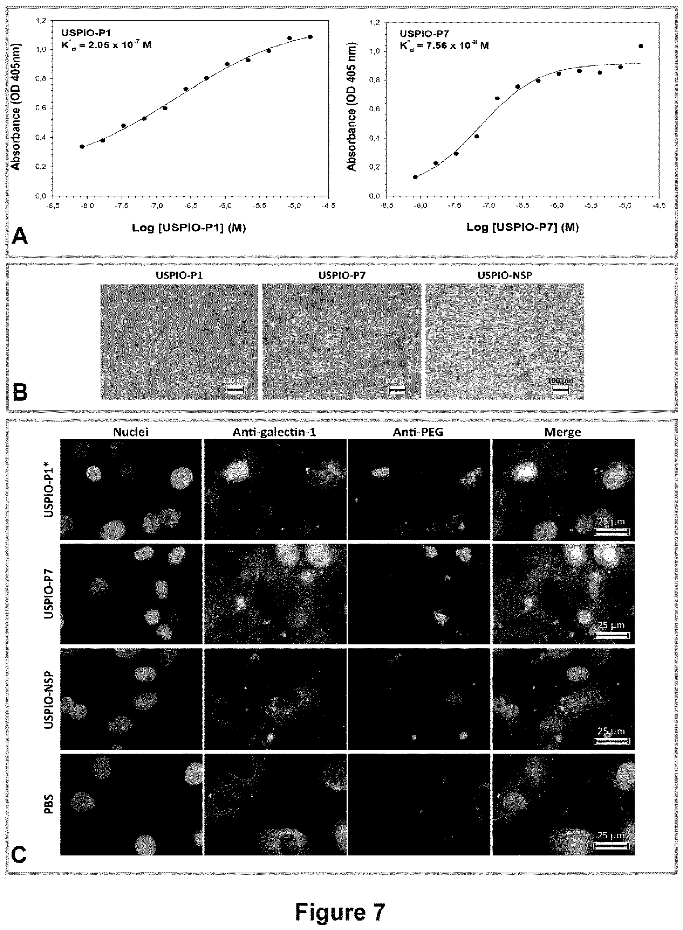

**Figure 7** shows the K*_{d} values of P1 and P7 coupled to Ultra-Small Particles of Iron Oxide (USPIO), USPIO-P1 and USPIO-P7 respectively (**A**), their binding to TPC-1 cells observed by Perls'-DAB staining (**B**) and co-localization of USPIO derivatives with gal-1 by immunofluorescence (**C**). Gal-1 is stained in green with Dylight 488 (shown in grayscale), USPIO derivatives are detected with anti-PEG antibody stained in red (shown in grayscale) by Texas red, and nuclei are stained in blue (shown in greyscale) with DAPI. USPIO-NSP is a control contrast agent functionalized with a non-specific peptide (NSP).

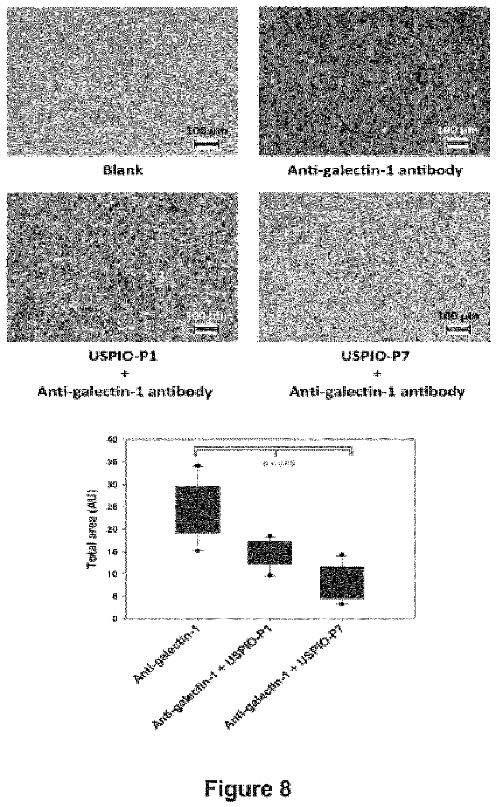

**Figure 8** shows the competition between USPIO-P1 or USPIO-P7 and anti-gal-1 antibody for the binding to gal-1 expressed by TPC-1 cells. Anti-gal-1 antibody bound to the target is stained in brown with DAB (shown in grayscale). The box-and-whisker plot represents the gal-1 staining in the absence or in the presence of USPIO derivatives as analyzed with ImageJ software. The difference between groups was analyzed by Mann-Whitney test.

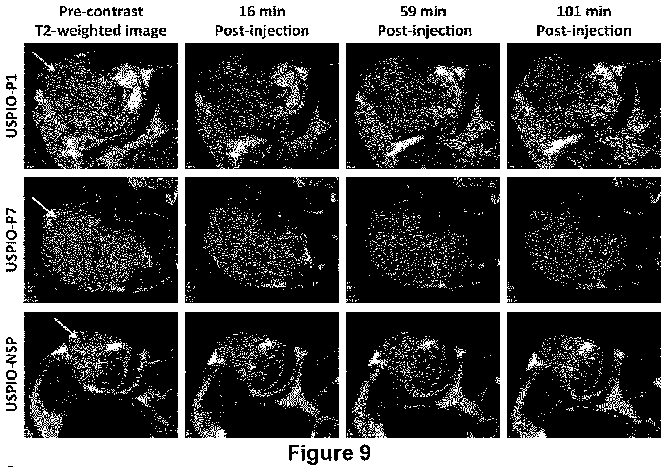

**Figure 9** shows *in vivo* Magnetic Resonance Imaging (MRI) acquired images of mice being injected with peptides according to an embodiment of the current invention. More specifically, Figure 9 shows MR images of TPC-1 tumors xenografted in athymic nude male mice before (pre-contrast) and after (post-contrast) the injection of either USPIO-P1, USPIO-P7 or USPIO-NSP.

### Detailed description of the invention

The present invention concerns gal-1 binding peptides and use thereof in visualization, diagnosis and treatment of thyroid malignancies.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

In a first aspect, the invention provides gal-1 binding peptides. Gal-1 is a protein which is known to be expressed in thyroid gland and over-expressed in thyroid cancer.

The term "peptide," as used herein and in the appended claims, refers to any compound containing two or more amino acid residues joined by an amide bond formed from the carboxyl group of one amino acid residue and the amino group of the adjacent amino acid residue. The amino acid residues may have the L-form as well as the D-form, and may be naturally occurring or synthetic, linear as well as cyclic. Also included within the term "peptide" as used herein and in the claims are polypeptides and peptide dimers which can be peptides linked C-terminus to N-terminus (tandem repeats) or peptides linked C-terminus to C-terminus or N-terminus to N-terminus (parallel repeats).

By preference, said peptide is a synthetic peptide. By the term "synthetic peptide" it is understood to refer to a peptide which has been artificially synthesized. Such synthesis methodologies are readily known in the art. The term "therapeutic peptide" as used herein denotes a bioactive peptide that has therapeutic utility.

Another method for the preparation of the peptides according to embodiments disclosed herein is the use of peptide mimetics. Mimetics are peptide-like molecules which mimic elements of protein or peptide secondary structure. The underlying rationale behind the use of peptide mimetics is that the peptide backbone of proteins exists chiefly to orient amino acid side chains in such a way as to facilitate molecular interactions. A peptide mimetic is expected to permit molecular interactions similar to the natural molecule.

The peptide according to the embodiments of the current invention may be purified. Generally, "purified" will refer to a protein or peptide composition which has been subjected to fractionation to remove various other components, and which composition substantially retains its expressed biological activity. Where the term "substantially purified" is used, this will refer to a composition in which the peptide forms the major component of the composition, such as constituting about 50% or more of the peptides in the composition.

Various methods for quantifying the degree of purification of the protein or peptide will be known to those of skill in the art in light of the present disclosure. These include, for example, determining the specific activity of an active fraction, or assessing the number of polypeptides within a fraction by SDS/PAGE analysis. Actual units used to represent activity will, of course, be dependent upon the particular assay technique chosen to follow the purification and whether or not the expressed protein or peptide exhibits a detectable activity.

Various techniques suitable for use in protein purification will be well known to those of skill in the art. These include, for example, precipitation with ammonium sulfate, PEG, antibodies and the like or by heat denaturation, followed by centrifugation; chromatography steps such as ion exchange, gel filtration, reverse phase, hydro xylapatite and affinity chromatography; isoelectric focusing; gel electrophoresis; and combinations of such and other techniques. As is generally known in the art, it is believed that the order of conducting the various purification steps may be changed, or that certain steps may be omitted, and still result in a suitable method for the preparation of a substantially purified protein or peptide.

There is no general requirement that the peptide always be provided in the most purified state. It is contemplated that less substantially purified products will have utility in certain embodiments. Partial purification may be accomplished by using fewer purification steps in combination, or by utilizing different forms of the same general purification scheme. For example, it is appreciated that a cation-exchange column chromatography performed utilizing an HPLC apparatus will generally result in a greater - fold purification than the same technique utilizing a low pressure chromatography system. Methods exhibiting a lower degree of relative purification may have advantages in total recovery of protein product, or in maintaining the activity of an expressed protein.

Gal-1 is by preference from mammal origin, more preferably mouse, rat or human gal-1.

By preference, the peptides will have a length of 6 to 25 amino acids, more preferably between 10 to 15 amino acids, even more preferably twelve amino acids.

In a further preferred embodiment, said peptides according to the current invention share at least 95%, more preferably at least 99% sequence identity to an amino acid sequence chosen from SEQ:ID n° 1 to 8, preferably SEQ:ID n° 1, 2 or SEQ:ID n°7. In another embodiment, said peptides have a sequence which differs maximally 3, more preferably maximally 2, even more preferably maximally 1 amino acid from one of the sequences chosen from SEQ:ID n° 1 to 8, preferably SEQ:ID n° 1, 2 or SEQ:ID n°7.

In another embodiment, said peptides have an amino acid sequence which encompasses one of the amino acid sequences chosen from SEQ:ID n° 1 to 8, or a sequence which has at least 95%, more preferably 99% sequence identity with on the amino acid sequences chosen from SEQ:ID n° 1 to 8, preferably SEQ:ID n° 1, 2 or SEQ:ID n°7.

The term "sequence identity" as used herein refers to the extent that sequences are identical on an amino acid-by-amino acid basis over a window of comparison. Thus, a "percentage of sequence identity" is calculated by comparing two optimally aligned sequences over the window of comparison, determining the number of positions at which the identical amino acid occurs in both sequences to yield the number of matched positions, dividing the number of matched positions by the total number of positions in the window of comparison (i.e., the window size), and multiplying the result by 100 to yield the percentage of sequence identity. A gap, i.e., a position in an alignment where a residue is present in one sequence but not in the other is regarded as a position with non-identical residues. Determining the percentage of sequence identity can be done manually, or by making use of computer programs that are available in the art. Examples of useful algorithms are PILEUP. Software for performing BLAST analyses is publicly available through the National Center for Biotechnology Information (http://www.ncbi.nlm.nih.gov/).

Amino acid sequence variants of a peptide contemplated herein may be substitutional, insertional or deletion variants. Deletion variants lack one or more residues of the peptide which may not be critical for function. Substitutional variants typically contain an alternative amino acid at one or more sites within the peptide and may be designed to modulate one or more properties of the polypeptide such as stability against proteolytic cleavage. Substitutions preferably are conservative, that is, one amino acid is replaced with one of similar size and charge. Conservative substitutions are well known in the art and include, for example, the changes of: alanine to glycine, valine or leucine; arginine to lysine; asparagine to glutamine; aspartate to glutamate; cysteine to methionine glutamine to asparagine; glutamate to aspartate; glycine to proline; histidine to asparagine or glutamine; isoleucine to leucine or valine; leucine to valine or isoleucine; lysine to arginine; phenylalanine to tyrosine, leucine or methionine; serine to threonine; threonine to serine; tryptophan to phenylalanine; tyrosine to tryptophan or phenylalanine; and valine to isoleucine or leucine.

In a most preferred embodiment, the sequence of said peptides have an amino acid sequence which is identical to a sequence chosen from SEQ:ID n° 1 to 8. More preferably, said sequence is SEQ:ID n° 1, 2 or SEQ:ID n°7.

Peptides with an amino acid sequence according to SEQ:ID n° 1 to 8 or a degree of sequence identity with said sequences as described above were found to bind to gal-1. Moreover, these peptides were confirmed to bind to gal-1 in thyroid tissue, hence making it suitable vehicles for locating to the thyroid gland or thyroid nodules, by preference malignant thyroid tissue gland, in a subject, such as a human patient. In a second aspect, these peptides were found to influence cell adhesion and cell signaling in the tumor cells, and could thus be used as therapeutic peptides, for the purpose preventing, delaying or reducing tumor growth, tumor size and/or metastasis.

As the peptides according to the current invention are found to locate to the thyroid or thyroid nodules, by preference malignant thyroid tissue they may be used to visualize the latter in a subject, e.g. a human patient. To that purpose the peptides will preferably be linked to a tracer or contrast agent, said tracer or agent is suitable for being used in a medical imaging methodology. Linkage may be to the C-terminus or N-terminus of the peptide or both.

For the purpose of the current invention, medical imaging is to be understood as a technique and process of creating visual representations of the interior of a body for clinical analysis and medical intervention, as well as visual representation of the function of some organs or tissues. Examples of medical imaging techniques which can be used in combination with the peptides according to the current invention include radiography, Tomography including Computed Tomography (CT), Positron Emission Tomography (PET), PET-CT, Single-Photon Emission Computed Tomography (SPECT), MRI and PET-MRI, Nuclear Medicine, fluorescent imaging or multimodal molecular imaging.

Said tracer or contrast agent may be directly linked or bound to an embodiment of the peptides as described above. In another embodiment, the peptide and tracer/contrast agent may be separated by a linker or spacer. Such linker/spacer may be flexible molecules or stretch molecules which ensure good functionality of both the peptide and the tracer/contrast agent by providing a distance (expressed in Angstrom) between the peptide and the tracer/contrast agent. The nature of the linker/spacer that is used may depend on the downstream application and/or the tracer/contrast agent. Example of spacers or linkers may include but are not limited to polyethylene glycol (PEG) linkers, i.e., 8-Amino-3,6-dioxaoctanoic acid, 12-amino-4,7,10-trioxadodecanoic acid, 15-amino-4,7,10,13-tetraoxapenta-decanoic acid, poly-glycine based linkers combined with serine [(Gly)4Ser], 6-aminocaproic acid, 5-aminovaleric acid, (2-aminoethoxy) acetic acid, different types of dendrimers, lysine and poly-lysine, etc.

Many tracers or contrast agents are suitable to be used in conjunction with the peptides according to the current invention, and the choice will largely depend on the downstream imaging technique that is used.

By preference, if MRI is employed, the tracer or contrast agent is chosen from superparamagnetic iron oxide (SPIO), ultrasmall superparamagnetic iron oxide (USPIO), or paramagnetic ion based contrast agent such as a gadolinium based contrast agent, a manganese based contrast agent, dysprosium based contrast agent, europium based contrast agent (may be used for chemical exchange saturation transfer (CEST) MRI, fluorescence imaging (FI) and multimodal FI-MRI) or a holmium based contrast agent.

When PET, PET-CT or SPECT is used, the tracer may be chosen from short half-life isotopes such as carbon-11 (¹¹C), nitrogen-13 (¹³N), oxygen-15 (¹⁵O), fluorine-18 (¹⁸F), gallium-68 (⁶⁸Ga), zirconium-89 (⁸⁹Zr), or rubidium-82 (⁸²Rb), copper-64 (⁶⁴Cu), copper-62 (⁶²Cu), copper-60 (⁶⁰Cu), iodine-124 (¹²⁴I), brom-76 (⁷⁶Br), indium-111 (¹¹¹In), iodine-123 (¹²³I), iodine-124 (¹²⁴I), iodine-125 (¹²⁵I), iodine-131 (¹³¹I), tellurium-125m (^{125m}Te), thulium-170 (¹⁷⁰Tm), lutetium-177 (¹⁷⁷Lu), Rhenium-186 (¹⁸⁶Re), Rhenium-188 (¹⁸⁸Re), astatine-211 (²¹¹At), galliun-67 (⁶⁷Ga), galliun-66 (⁶⁶Ga), galliun-68 (⁶⁸Ga), technetium-99m (99mTc), yttrium-86 (⁸⁶Y), zirconium-89 (⁸⁹Zr), cobalt-55 (⁵⁵Co), strontium-83, (⁸³Sr), manganese-51 (⁵¹Mn), manganese-52 (⁵²Mn), fer-52 (⁵²Fe).

For CT, contrast generating elements such as iodine, gold, bismuth, bromine, tantalum, platinum, ytterbium, yttrium, gadolinium, tungsten may be sued. Here, a core is coated with a polymer, lipid, protein, silica or other compounds that yield solubility in biological media and biocompatibility. The coating can be modified in various ways to include targeting moieties (antibodies, proteins, peptides, aptamers and so forth).

For optical imaging, suitable fluorescent contrast agents are 5-aminolevulinic acid (5-ALA) and indocyanine green (ICG) dyes. Conjugation of ICG to targeting moieties, such as peptides and antibodies, requires further modifications of the dye molecule. At present, there are few available dyes, such as Cy5.5 (GE Healthcare, Piscataway, NJ), DyLight 680 to 800 (Thermo Fisher Scientific Inc., Rockford, IL), and IRDye 800CW (LI-COR Biosciences, Lincoln, NE), that are excitable in the NIR wavelength window and functionalized for conjugation to various targeting molecules. The most common modifications for NIR dyes are N-hydroxysuccinimide (NHS) ester and maleimide, which permit conjugation to tumor targeting antibodies, peptide ligands, and small molecules with primary aliphatic amines.

For multimodal molecular imaging hybrid molecules containing two (or more) imaging probes are used such as for instance iron oxide nanoparticles coupled with a fluorescent dye, or paramagnetic and fluorescent dendrimers, or paramagnetic and radioactive dendrimers, or paramagnetic and radioactive chelates.

By linkage of a tracer/contrast agent of choice to one of the embodiments of the peptides as described above, malignancies of the thyroid gland or thyroid nodules, preferably papillary thyroid tumors, can be visualized.

The peptides of the current invention have an apparent dissociation constant K*_{d} for gal-1 which is preferably between 0.5 x 10⁻⁵ M and 5 x 10⁻⁵ M, more preferably between 1 x 10⁻⁵ M and 3.5 x 10⁻⁵ M. When fused to a tracer or contrast agent like USPIO, SPIO or dendrimers, the K*_{d} of the peptides will preferably be between 10 x 10⁻⁸ M and 0.5 x 10⁻⁶ M, more preferably between 8 x 10⁻⁸ M and 2 x 10⁻⁷ M.

Based on K*_{d}, an affinity constant Ka (Ka = 1/K*_{d}) may be determined.

The peptides have a preferred *in vivo* half-life between 15 minutes and 5 hours, more preferably between 2 and 4 hours. This half-life was found to be sufficient for the imaging purpose of the current invention, after which the peptide is cleared out of the body.

The half-life may differ based on the choice of linker or contrast agent. Peptides grafted to USPIO will have a half-life ranging between 2 and 5 hours. Simple chelates such as DTPA or DOTA may have a half-life ranging of about 15-30 min. When the chelate is grafted to a peptide, the half-life is slightly prolonged to 30-45 min. Dendrimers on the other hand will have long half-lives, typically ranging from 3 - 15 hours or longer.

It will be clear to the skilled person that the current invention also extends to mimetics which mimic the peptides of the current invention.

The present invention also pertains to a diagnostic or pharmaceutical composition for cancer or malignancies of the thyroid gland, comprising one or more peptides according the embodiments as described above.

Embodiments herein provide for administration of compositions to subjects in a biologically compatible form suitable for pharmaceutical administration *in vivo.* By "biologically compatible form suitable for administration *in vivo*" is meant a form of the active agent (e.g., pharmaceutical chemical, protein, gene, antibody etc. of the embodiments) to be administered in which any toxic effects are outweighed by the diagnostic or therapeutic effects of the active agent. Administration of an active amount of the compositions according to the current invention is defined as an amount effective, at dosages and for periods of time necessary to achieve the desired result, whether it may be for imaging or therapeutic reasons.

For example, an active amount of a compound may vary according to factors such as the disease state, age, sex, and weight of the individual, and the ability of the peptides to elicit a desired response in the individual. Dosage regimen may be adjusted to provide the optimum therapeutic response.

Diagnostic or pharmaceutical compositions containing a peptide of the current invention, or analog thereof, or a functional derivative thereof (e.g., a mimetic of said peptides) may be administered to a subject, for example by subcutaneous, intravenous, intracardiac, intracoronary, intramuscular, by oral administration (formulated as solutions, lyophilized powders, capsules, tablets, liposomes, and the like), by inhalation, transdermal application, intravaginal application, topical application, intranasal or rectal administration. Depending on the route of administration, the active compound may be coated in a material to protect the active agent from the degradation by enzymes, acids and other natural conditions that may inactivate the compound. In a preferred embodiment, the composition may be administered intravenously.

The active agent (in the current case the peptide) may be administered to a subject in an appropriate carrier or diluent, coadministered with enzyme inhibitors or in an appropriate carrier such as liposomes. The term "pharmaceutically acceptable carrier" as used herein is intended to include diluents such as saline and aqueous buffer solutions. It may be necessary to coat the compound with, or coadminister the compound with, a material to prevent its inactivation. The active agent may also be administered parenterally or intraperitoneally. Dispersions can also be prepared in glycerol, liquid polyethylene glycols, and mixtures thereof and in oils. Under ordinary conditions of storage and use, these preparations may contain a preservative to prevent the growth of microorganisms.

Pharmaceutical compositions suitable for injectable use may be administered by means known in the art. For example, sterile aqueous solutions (where water soluble) or dispersions and sterile powders for the extemporaneous preparation of sterile injectable solutions or dispersion may be used.

Sterile injectable solutions can be prepared by incorporating the active agent in the required amount in an appropriate solvent with one or a combination of ingredients enumerated above, as required, followed by filtered sterilization.

Aqueous compositions can include an effective amount of the active agent, being one or more peptides according to the current invention dissolved or dispersed in a pharmaceutically acceptable carrier or aqueous medium. Compounds and biological materials disclosed herein can be purified by means known in the art. Solutions of the active compounds as free-base or pharmacologically acceptable salts can be prepared in water suitably mixed with a surfactant.

Upon formulation, solutions will be administered in a manner compatible with the dosage formulation and in such amount as is effective. The formulations are easily administered in a variety of dosage forms, such as the type of injectable solutions described above. These particular aqueous solutions are especially suitable for intravenous, intramuscular, subcutaneous and intraperitoneal administration.

Active agents may be formulated within a mixture to comprise about 0.0001 to 1.0 milligrams, or about 0.001 to 0.1 milligrams, or about 0.1 to 5.0 mg per dose or even about 1 to 10 gram per dose, depending on whether or not a linker or contrast agent is present (e.g. not necessary for therapeutic reasons) and if such linker or agent is present, the nature of the agent. If the peptide is used as such (e.g. for therapeutic reasons) the chosen dose may lie between 1 mg/dose to 15 g/dose, more preferably 1 g to 10 g/dose.

When the composition is used for therapeutic purpose, a single dose or multiple doses can also be administered on an appropriate schedule for a predetermined condition such as daily, bi-weekly, weekly, bimonthly etc. Pharmaceutical compositions are administered in an amount, and with a frequency, that is effective to modulate side effects. The precise dosage and duration of treatment may be determined empirically using known testing protocols or by testing the compositions in model systems known in the art and extrapolating therefrom. Dosages may also vary with the severity of the condition. In certain embodiments, the composition range can be between 10 and 75 mg/kg introduced daily or weekly to a subject. A therapeutically effective amount can be also measured in molar concentrations and can range between about 10 nmol to about 100 µmol of peptide per kg body weight of said subject, more preferably between 20 nmol to 20 µmol of peptide per kg body weight of said subject.

The current invention equally pertains to a kit having a composition comprising one or more peptides disclosed herein of use to visualize the thyroid gland or thyroid nodules, by preference the malignant thyroid tissue in a subject and/or to detect metastasis in a subject. Other embodiments concern kits of use to assess completion of tumor/cancer cell removal of by a health professional before, during or after a surgical procedure.

In another embodiment, said kit is used for therapeutic purpose, whereby tumor growth or metastasis is prevented, reduced and/or delayed.

In an aspect, the current invention also pertains to a method for imaging gal-1 expressing tissues and/or organs in a subject, whereby a gal-1 binding peptide according to any one of the above mentioned embodiments or a composition is administered to said subject. By preference, said subject is human. The method could be used to visualize any tissue or organ which is expressing gal-1, but is preferably useful for visualizing thyroid cancer, which is known to overexpress gal-1.

As explained above, various imaging methods can be combined with the subject of the current invention of which MRI and nuclear imaging techniques are particularly useful. For MRI, the peptide will preferably be administered to said subject in a dose of between 10 nmol of peptide per kg body weight of said subject and 100 µmol of peptide per kg body weight of said subject. When a nuclear imaging technique is used, the peptide will preferably be administered to said subject in a dose of 1 nmol of peptide per kg body weight of said subject and 1 µmol of peptide per kg body weight of said subject.

In another aspect, the current invention also pertains to a method for providing information for thyroid cancer diagnosis or malignancies of the thyroid gland, comprising the steps of:
a) administering a composition including the gal-1 binding peptide according to any of the above described embodiments to a patient suspected of having thyroid cancer; and
b) imaging the thyroid region by means of a medical imaging methodology.

The method is particularly useful in collecting information on thyroid cancers of the group of papillary thyroid cancer, follicular thyroid cancer, or anaplastic thyroid cancer.

In a final aspect, the current invention pertains to the peptides according to one or more embodiments as described above, for therapeutic use, especially treatment of cancer or malignancies or the prevention or inhibition of cancer growth, cell proliferation and/or metastasis. Said cancer is by preference thyroid cancer. The method of the present invention can be advantageously combined with at least one additional treatment method, including but not limited to, chemotherapy, radiation therapy, or any other therapy known to those of skill in the art for the treatment and management of proliferation disorders such as cancer.

Therapeutic application of the peptides and compositions according to the current invention comprising them can be accomplished by any suitable therapeutic method and technique presently or prospectively known to those skilled in the art. Further, the peptides of the invention can be used as starting materials or intermediates for the preparation of other useful compounds and compositions.

The present invention will be now described in more details, referring to examples that are not limitative.

### Examples

### Material and methods

### 1. Phage display screening

### 1.1. Panning of the phage display library against human gal-1

In order to identify gal-1 targeted peptides, two distinct phage display experiments (PDE1 and PDE2) were performed. For both of them, a linear 12-mer random peptide phage display library (PhD-12, New England Biolabs Inc. Bioké, Leiden, The Netherlands) was screened (six rounds for PDE1, three rounds for PD2) against human gal-1 (Peprotech, London, UK for PDE1; Creative Biomart, New York, USA for PDE2), which was immobilized on a 96-well ELISA plate at a concentration of 100 µg/mL. The preselection step was performed by incubating the phage library with bovine serum albumin (BSA, 1 µg/mL, Sigma-Aldrich, Diegem, Belgium) for PDE1 and with a mix of human serum albumin (HSA) and collagen (both at 100 µg/mL; Sigma-Aldrich) for PDE2.

The target and the control proteins are immobilized in a 96-wells ELISA plate (Greiner BioOne) overnight at 4°C in a basic buffer (NaHCO₃ 0,1 M, pH 8.6). Subsequent the immobilization, the wells were blocked one hour with Protein-Free Blocking Buffer (Thermo Fisher Scientific, Belgium). The percentage of Tween-20 in the rinsing buffer (TBS) is also increased along the rounds (Table 1, first experiment: 0.1 - 0.3 - 0.5 - 1 - 2 - 4 %; second experiment: 0.5 - 1 - 1.5%). After rinsing, the library during the first round or the output for the next rounds (2 x10¹¹ phages) was incubated with the control protein. The time for the preselection step is modified along the rounds (first experiment: 80 - 100 - 120 - 140 - 160 -180 min; second experiment: 80 - 100 - 120 min). Then the phages are transferred into the target well during a decreasing time along the rounds (first experiment: 180 - 150 - 120 - 90 - 60 - 30 min; second experiment: 90 - 60 - 30 min). The target well is then rinsed with a TBS-Tween-20 solution and the phages bound are recovered by an elution with a Glycine-HCl-BSA buffer during 20 minutes. Finally, the solution is neutralized with Tris-HCl buffer pH 9.1 and the final solution corresponds to the output.

**Table 1 Parameters of phage display experiments**

| **Phage display** | **Phage library** | **Target** | **% Tween-20** | **Preselection step** | **Time on target** | **Number of rounds** |
|---|---|---|---|---|---|---|
| **1** | Linear 12-mer peptides | Gal-1 (Peprotech): 100 µg/mL) | 0.1%, 0.3%, 0.5%, 1%, 2%, 4% | On BSA: 80 min, 100 min, 120 min, 140 min, 160 min, 180 min | 180 min, 150 min, 120 min 90 min, 60 min, 30 min | 6 |
| **2** | Linear 12-mer peptides | Gal-1 (Creative Biomart): 100 µg/mL) | 0.5%, 1%, 1.5% | On HSA+Collagen: 80 min, 100 min, 120 min | 90 min, 60 min, 30 min | 3 |

The outputs are amplified between each round by Escherichia coli infection (ER2738 strain, New England Biolabs), at mid-log phase. The bacteria are cultured in Luria Broth medium supplemented with Tetracycline (Sigma-Aldrich), at 37°C under agitation (160 rpm) during 4.5 hours. The culture is then centrifuged at 10 000 RPM in order to collect the supernatant. This one is subsequently precipitated (overnight, 4°C) with Polyethylene glycol 8000 - NaCl 2.5M (1/6) and then centrifuged 20 minutes at 10 000 RPM. The pellet is recovered and suspended in 50 - 100 µL TBS. Once the best output is selected according to its constant affinity towards the target, 50 phages clones are isolated and chosen arbitrarily. Ten dilutions of the output are prepared in LB and are added to the E. coli culture at mid-log phase during 5 minutes. The bacteria infected are then mixed with a melted agarose solution that is finally deposited on an LB/IPTG/Xgal plate. The clones are finally selected one by one and added to an E. coli culture for amplification.

### 1.2. Sequencing of the selected phage clones

DNA from the selected clones was extracted using the phenol/chloroform method. DNA sequencing was performed to decipher the peptide sequence fused to pIII protein sequence of the bacteriophage (Beckman Coulter Genomics, Grenoble, France). The primer used is composed of 20 bases (5'-CCC TCA TAG TTA GCG TAA CG-3') and anneals to 96 bases downstream of the inserted sequence.

The bioinformatics webserver (http://www.bioinformatics.org/sms/index.html) was used to obtain the reversed and complementary sequence of the anti-sense DNA that was then translated.

### 1.3. Gal-1 binding of the isolated phage clones

Human gal-1 (Peprotech) and control proteins (HSA and collagen) were immobilized on 96-well ELISA plates at a concentration of 10 µg/mL in NaHCO₃ 0.1 M, pH 8.6, overnight at 4°C. The wells were then blocked with Protein-Free Blocking Buffer (PFBB, Thermo Fisher Scientific, p/a Perbio Science BVBA, Aalst, Belgium), 1 hour at room temperature. Fifty clones issued from each phage display experiment were first screened at one concentration (5 x 10¹¹ phages/100 µL for PDE1; 10¹¹ phages/100 µL for PDE2) prepared in Tris buffered saline (TBS: 50 mM Tris-HCl, 150 mM NaCl, pH 7.5) supplemented with 0.5% Tween-20 (TBST). The clones were incubated for 2 hours at room temperature, under mild agitation. To determine the apparent dissociation constant (K*_{d}) of the selected clones, ten serial dilutions of phages (generally ranging from 5x10¹² to 10⁷ phages/100 µL) were incubated with gal-1 or control proteins in the same conditions as for the first clones' screening. The bound phages were detected by an incubation (1 hour, room temperature) with horseradish peroxidase (HRP)-conjugated anti-M13 monoclonal antibody (Amersham Pharmacia Biotech Benelux, Roosendaal, The Netherlands) diluted 1:5000 in TBS supplemented with 0.5% BSA. After each incubation step, the wells were rinsed with an automated microplate washer (Beckman Coulter, Analis, Suarlée, Belgium). Finally, the wells were filled with 200 µL/well of 2,2'-azino-bis(3-ethylbenzothiazoline-6-sulphonic acid (ABTS, 22 mg) diammonium salt (Sigma-Aldrich) solution prepared in 50 mM sodium citrate pH 4.0 completed with 0.05% H₂O₂. The optical density (OD) was measured (405 nm, differential filter: 630 nm) using a microplate reader (StatFax-2100, Awareness Technology, Fisher Bioblock Scientific, Tournai, Belgium).

### 2. Characterization of the selected peptides

### 2.1. Estimation of K*_{d}

The gal-1 targeted and control peptides (negative control peptide, called NCP; scrambled peptide 1, called P1Scr; non-specific peptide, called NSP) were synthesized as biotinylated or not biotinylated 8-amino-3,6-dioxaoctanoyl derivatives (Eurogentec, Seraing, Belgium; PolyPeptide Laboratories, Strasbourg, France). To evaluate their K*_{d}, ten serial dilutions of biotinylated peptides were incubated with gal-1 immobilized on 96-well ELISA plates as described above. The wells were blocked with PFBB and the bound peptides were detected with 1 µg/mL of anti-biotin antibody made in goat and 0.2 µg/mL of anti-goat IgG antibody made in horse (both from Vector Labconsult, Brussels, Belgium). The staining reaction was developed as described above with ABTS/H₂O₂, and OD₄₀₅ was measured with a SpectraMax M2 microplate reader (Molecular Devices, Workingham, Berks, UK).

### 2.2. Circular dichroism studies and bioinformatics studies

Non-biotinylated peptides dissolved in distilled water at 1 mM were used for structure analysis. Circular Dichroism (CD) spectra were recorded using a ChirascanTM Plus CD Spectrometer (Applied Photophysics Inc., Leatherhead, United Kingdom). The measurements were carried out using 1 mm quartz Suprasil cells from Hellma Analytics (Müllheim, Germany). The spectra were recorded between 180 and 350 nm, with a bandwidth of 0.5 nm, time per point 2 s and three repetitions, both at 20 °C and 37 °C. The CD RMS (Root Mean Square) noise is around 0.03 mdeg at 180 nm. The aqueous solution reference spectra were used as baselines and were automatically subtracted from the CD spectra of the samples.

The complementary bioinformatics studies were performed on the sequences of both peptides, using the InterPro webserver for domain scanning, the Motif Scan webserver for motif scanning (http://myhits.isb-sib.ch/cgi-bin/motif_scan), and the DisEMBL 1.5 webserver to predict the intrinsic disorder of both peptides.

### 2.3. Docking studies of the binding modes of peptides with gal-1

In order to predict the binding modes of the peptides with gal-1, docking calculations were performed. Among docking algorithms, the CABS-dock method was chosen for its ability to perform docking between small peptides and proteins in a blind mode, i.e., without prior knowledge of the binding site. Additionally, CABS-dock does not require peptide structural information, the sequence being sufficient. Peptides are considered as fully flexible. The flexibility of the ligand as well as the fluctuations of the target backbone are essential as recently proved when revealing large-scale conformational changes that follow ligand binding. The CABS-dock method, based on the CABS coarse-grained protein models performs Replica Exchange Monte-Carlo (REMC) simulations for an efficient sampling of the docking simulations.

The crystallographic structure of gal-1 (PDB ID: 3W58) has been chosen in regards to its unbound state, its good resolution (1.58 Å) and the absence of important mutations. Peptide secondary structures were predicted within CABS-dock with PSIPRED prediction. In the docking calculations, 100 Monte-Carlo (MC) simulations cycles were performed.

Totally blind predictions of the ligand poses were considered, without specifying (un)likely binding regions on the proteins, neither marking specific flexible regions. 1,000 models were generated per docking trajectory. Within CABS-dock, a structural clustering was operated on the 1,000 top poses of the entire set of generated docking solutions, giving at the end 10 clusters. From these clusters, 10 consensus models were generated. For each peptide, the best consensus solution was used for inspection and further studies within PyMol. For this, fine-graining of the selected consensus models were performed within CABS-dock to allow all-atom resolution analysis.

### 2.4. Gal-1 expression and binding of peptides to human biopsies of thyroid cancer

Immunohistochemistry evaluation was performed on 5 µm thick sections mounted on silane-coated glass slides. The paraffin-embedded tissue specimens were deparaffinized and rehydrated. After unmasking the epitopes in citrate buffer pH 6.0, the endogenous peroxidase activity was blocked with 0.7% H₂O₂. Sections were then blocked with streptavidin - biotin blocking kit (Vector Labconsult) and with 0.5% casein (Sigma-Aldrich).

To evaluate the gal-1 expression by tumor biopsies, they were incubated overnight at 4°C with 10 µg/mL of anti-human gal-1 antibody made in rabbit (Santa Cruz, Heidelberg, Germany), followed by one hour incubation with 30 µg/mL of biotinylated anti-rabbit antibody made in goat (Vector Labconsult) and then with Vectastain ABC kit (Vector Labconsult). The sections were subsequently treated for 5 minutes with 50 mM Tris-HCl pH 7.4, followed by their staining with 0.05% 3,3'-Diaminobenzidine (DAB) tetrachlorhydrate (Sigma-Aldrich) completed with 0.02% H₂O₂. The sections were counterstained with Mayer's Hemalun solution (VWR International, Leuven, Belgium) and were finally mounted with a synthetic medium (Leica Microsystems, Groot Bijgaarden, Belgium).

The binding of biotinylated peptides to tumor biopsies was validated by using a similar protocol. However, no epitope unmasking was performed, while casein blocking was replaced by PFBB. Endogenous peroxidases were blocked with 1% H₂O₂. The biotinylated peptides (stock prepared in distilled water) were diluted in TBS at a concentration of 20 µM and incubated with tumor biopsies overnight at 4 °C. The bound peptides were then detected with 5 µg/mL of anti-biotin antibody made in goat (Vector Labconsult) and with 1 µg/mL of biotinylated anti-goat IgG made in horse (both from Vector Labconsult) both diluted in TBS completed with 0.5% BSA (5 µg/mL). After incubation with Vectastain ABC kit (Vector Labconsult), the staining with DAB/H₂O₂ and Hemalun counterstaining were performed as described above.

### 2.5. Masson's Trichrome staining of human biopsy sections

The Sigma-Aldrich kit for Masson's Trichrome staining was used according to the kit instructions. After having deparaffinized and rehydrated the paraffin-embedded tissue specimens, the histological sections were treated first with Bouin's solution in order to intensify the final staining. Nuclei were stained in black with Weigert's iron hematoxylin, while cytoplasm was stained in red with Biebrich solution. Finally, the collagen was stained in blue with aniline blue after treatment with phosphotungstic and phosphomolybdic acid. The tissue sections were then rinsed in acetic acid and distilled water and finally mounted after dehydration.

### 2.6. Semi-quantitative analysis of gal-1 expression and of peptide binding to human biopsies

Gal-1 staining on immunohistochemistry microphotographs (DM2000 Leica microscope equipped with a DFC 425C camera, Leica Microsystems) was semi-quantitatively analyzed by ImageJ software (National Institutes of Health, USA). The expression of gal-1 was compared in 5 cases of healthy thyroid, 2 cases of inflammatory thyroid (patients with goiter), 6 cases of thyroid adenoma, 6 cases of papillary thyroid cancer, 4 cases of follicular variant of papillary thyroid cancer and 6 cases of anaplastic cancer. The binding of peptides 1 and 7 was compared in 5 cases of healthy thyroid, 2 cases of inflammatory thyroid, 6 cases of thyroid adenoma, 3 cases of papillary thyroid cancer, 3 cases of follicular thyroid cancer and 6 cases of anaplastic thyroid cancer. The total area occupied by brown stained pixels was determined after thresholding the background staining on blank histological slices, where the primary antibody was excluded. The total stained area is then automatically generated after pixel segmentation.

### 2.7. Co-localization of peptides with gal-1 expressed by TPC-1 cells

The human thyroid papillary carcinoma-1 (TPC-1) cell line was grown at 37 °C and 5% CO₂ in RPMI 1640 medium with glutamine, supplemented with 10% fetal bovine serum and 1% penicillin/streptomycin (all from Life Technologies, Gent, Belgium).

For co-localization tests, TPC-1 cells (50,000 cells/well) were grown in 4-wells culture glass slides (Merck, Overijse, Belgium) until confluence. After fixation with 4% formalin (Sigma-Aldrich), cells were blocked with streptavidin-biotin blocking kit (Vector Labconsult). Biotinylated peptides were then incubated overnight at a concentration of 30 µM, at 4°C. Cells were then incubated for two hours with 20 µg/mL of anti-human gal-1 antibody made in rabbit (Santa Cruz), followed by one-hour incubation with 10 µg/mL of anti-biotin antibody made in goat (Vector Labconsult). Peptides were then stained in red with 30 µg/mL of Dylight 594 horse anti-goat antibody, whereas gal-1 was stained in green with 15 µg/mL of Dylight 488 horse anti-rabbit antibody (both from Vector Labconsult). Fluorescent secondary antibodies were diluted in phosphate buffer pH 7.8 supplemented with 0.5% BSA and incubated with cells for 1 hour at room temperature. Cells were then mounted with 4',6-diamidino-2-phenylindole (DAPI) mounting medium (Vector Labconsult).

### 2.8. Cell adhesion assay

Gal-1 (Peprotech) was immobilized on 96-well culture plates at 20 µg/mL overnight at 4°C. After blocking with 0.1% BSA prepared in phosphate buffered saline (PBS, per liter: 8 g NaCl, 0.2 g KCl, 2.31 g Na₂HPO₄ × 12 H₂O 0.2 g KH₂PO₄, pH 7.4), non-biotinylated peptides (P1, P7 or P1Scr) diluted in RPMI 1640 culture medium at 0.5 µM or 50 µM were pre-incubated with gal-1 for 2 hours. In the meanwhile, TPC-1 cells (10⁵ cells/well) were pre-incubated with the desired concentration of peptides in RPMI 1640 culture medium for 30 minutes before seeding them into the wells. The cells were then incubated (37 °C, 5% CO₂) with gal-1 immobilized on wells for one hour. The negative control cells were incubated with RPMI 1640 culture medium alone. After washing steps and plate centrifugation between them, cells were fixed 15 minutes with buffered 4% formalin (from Sigma-Aldrich) and washed before staining with 1% crystal violet. Wells were rinsed and crystal violet was solubilized with dimethyl sulfoxide (DMSO, Sigma-Aldrich) before measuring absorbance at 570 nm (differential filter at 630 nm) with a microplate reader. The percentage of adherent cells was obtained by calculating the percentage ratio of OD measured for TPC-1 incubated with peptides over the OD of negative control cells.

The results prove that binding of the peptides to the target is specific but equally shows the therapeutic value of the peptides in treating thyroid cancer.

### 2.9. Evaluation of apoptosis by immunodetection of active caspase 3

TPC-1 cells seeded on 4-wells culture glass slides at a cell density of 30,000 cells per well were treated during 24 hours with 0.5 µM of non-biotinylated peptides (P1, P7 or P1Scr) or with 2 µM of camptothecin (MP Biomedical Europe SA, Bruxelles, Belgium) as positive control. After cell fixation with frozen methanol at -20 °C, they were blocked with PFBB before overnight incubation with 15 µg/mL of monoclonal anti-active caspase 3 antibody made in rabbit, (Fischer Scientific, Brussels, Belgium). Active caspase-3 was revealed with 20 µg/mL of Dylight 488 anti-rabbit antibody (made in horse, Vector Labconsult) and nuclei were stained with DAPI (Vectorlab).

### 3. Conjugation of peptides to USPIO and characterization of USPIO derivatives

Iron oxide nanoparticles were coated with an organic polysiloxane exhibiting carboxylic acid functions (3-(triethoxysilyl)propyl succinic anhydride; ABCR, Germany). Peptides were synthesized (Polypeptide; Eurogentec) with an 8-amino-3,6-dioxaoctanoyl moiety attached to their N-terminus. As previously described (Stanicki et al. 2014), peptides were conjugated to Fe₂O₃ surface by using N-(3-dimethylaminopropyl)-N'-ethylcarbodiimide hydrochloride (TCI, Tokyo, Japan) as a coupling agent. Then, O-(2-aminoethyl)-O'-methylpolyethyleneglycol chains (average molecular weight: 750 g/mol; Sigma Aldrich) were conjugated to saturate free carboxyl groups and to increase the stealth of vectorized nanoparticles. An ultrafiltration step was performed through a 100 kDa-membrane (Millipore, Overijse, Belgium) to obtain purified USPIO derivatives. Their hydrodynamic diameter was measured by photon correlation spectroscopy (PCS, Brookhaven system BI-160 (New York, USA) equipped with a He-Ne laser (=633 nm, 35 mW)) after 2 and 24 hours of incubation at room temperature in water (about 53 nm for USPIO-P1 and 43 nm for USPIO-P7) and in PBS (about 56 nm for USPIO-P1 and 36 nm for USPIO-P7).

The K*_{d} of USPIO-P1 and USPIO-P7 for the binding to gal-1 was determined by ELISA. The twelve concentrations of the titration curves ranged from 1.90·10⁻¹ M to 9.28·10⁻⁵ M of iron (1.72·10⁻⁵ to 8.43·10⁻⁹ mole particles per liter by assuming ∼11,000 iron atoms per particle). An anti-PEG monoclonal antibody (made in rabbit, Abcam, Cambridge, UK) was used at 2 µg/mL to detect them. Subsequently, the biotinylated anti-rabbit antibody (5 µg/mL, made in goat) and Vectastain ABC kit (both from Vector Labconsult) were employed before revelation reaction as described above.

### 3.1. Binding of USPIO derivatives to thyroid cancer cells assessed by Perls'-DAB staining

TPC-1 cells (3·10⁵ cells/slide) were grown on slides coated with 200 µg/mL of collagen (Sigma-Aldrich) until confluence in 6-well plates. After fixation with 4% buffered formalin, background was reduced by incubating cells with 1% H₂O₂. Cells were then incubated with 2 mM iron of USPIO-P1, USPIO-P7 or USPIO-NSP for 2 hours at room temperature. After 30 minutes of incubation with Perls' solution (5% potassium ferrocyanure, 2% HCl, 1:1), cells were pre-treated for 10 minutes with 0.05% DAB in PBS, followed by an incubation for 9 minutes with 0.05% DAB supplemented with 0.033% H₂O₂ prepared in PBS. The cells were counterstained with Luxol fast blue, and slides were finally mounted in a permanent medium after dehydration baths.

### 3.2. Immunofluorescence colocalization of USPIO derivatives with gal-1 expressed by TPC-1 cells

TPC-1 cells were seeded on 4-wells culture glass slides (30,000 cells/well) and grown until confluence. After fixation with 4% buffered formalin, cells were blocked during one hour with PFBB. An overnight incubation with 2 mM iron of USPIO (USPIO-P1, USPIO-P7, USPIO-NSP) or PBS was then performed. The cells were then incubated for two hours with 20 µg/mL of anti-gal-1 antibody (made in rabbit, Santa Cruz), followed by two hours with 8 µg/mL of anti-PEG antibody (made in rat, Abcam). Nanoparticles were detected with 20 µg/mL of Texas Red goat anti-rat antibody and the target with Dylight 488 horse anti-rabbit antibody (both from Vector Labconsult). The antibodies were diluted in 10 mM phosphate buffer pH 7.8 completed with 0.5 % BSA.

### 4. Competition assay between USPIO derivatives and anti-gal-1 antibody revealed by immunodetection of gal-1

TPC-1 cells (5·10⁴ cells/slide) were grown on slides coated with 200 µg/mL of collagen (Sigma-Aldrich) until confluence in 12-well plates. After fixation with 4% buffered formalin, background was reduced by incubating cells with 1% H₂O₂. Cells were then blocked with streptavidin-biotin blocking kit (Vector Labconsult). They were first incubated with 5 mM iron of USPIO-P1 or USPIO-P7 for 30 minutes at room temperature, whereas control cells were incubated with PBS. Subsequently, cells were incubated with a solution containing 5 mM iron of USPIO-P1 or USPIO-P7 and 10 µg/mL of anti-gal-1 antibody made in rabbit (antibodies-online GmbH, Aachen, Germany) for 90 minutes at room temperature; control cells were incubated with anti-gal-1 antibody alone. Then, the biotinylated anti-rabbit antibody (30 µg/mL, made in goat) and Vectastain ABC kit (both from Vector Labconsult) were employed before revelation reaction with DAB as described above. The cells were finally counterstained with Luxol fast blue, and slides were finally mounted in a permanent medium after dehydration baths.

### 5. MTT cytotoxicity assay

The cytotoxicity assays were performed on HepaRG human hepatocytes (Life Technologies) and on TPC-1 cell lines. HepaRG cells were cultivated (37 °C, 5% CO₂) in Williams'E medium supplemented with 1% glutaMAX and 13% Thaw, Plate & General Purpose Supplement (TPGPS); for toxicity tests, TPGPS was replaced by HepaRG Tox medium supplement (all from Life Technologies). TPC-1 cells were grown as described above.

For toxicity assays, cells were seeded (1.4·10⁴ cells/well) in 96-well culture plates (Life Technologies). Once attained confluence, TPC-1 or HepaRG cells were incubated for 2 or 24 hours with peptides or USPIO derivatives diluted in culture medium at the desired concentrations. The negative control cells were incubated in culture medium alone and the tests were performed in triplicate. After rinsing with PBS, the cells were incubated for 3 hours at 37 °C with MTT [3-(4,5-dimethylthiazol-2-yl)-2,5-diphenyl tetrazolium bromide] solution (In vitro toxicology assay kit MTT based, Sigma-Aldrich) diluted in culture medium (1:10). The resulting formazan crystals were dissolved with DMSO. The absorbance was measured at 570 nm with a differential filter at 690 nm. The percentage of viable cells was calculated by the ratio of OD of treated cells over the OD of negative control cells.

### 6. Statistical analysis

The results are expressed as average ± standard deviation (SD) and the differences between experimental groups were compared by one-way ANOVA using the SigmaPlot 11.0 software. The results obtained by ImageJ treatment were analyzed using the Mann-Whitney test. P-value lower than 0.05 indicates a significant difference between groups.

### 7. In vivo imaging

The *in vivo* experiments were approved by the Ethical Committee of the University of Mons, Belgium and complied with the Directive 2010/63/EU. After anesthesia with pentobarbital (50 mg/kg b.wt., i.p.) and analgesia with buprenorphine (0.05 mg/kg b.wt., s.c.), male athymic nude mice (Envigo, Venray, The Netherlands) were xenografted subcutaneously in the thigh or orthotopically at the neck with 7 x 10⁶ TPC-1 cells diluted in culture medium using an insulin syringe with a BD Microlance 3 25G needle (0,5 x 25 mm). Two or three weeks later, the mice were anesthetized with pentobarbital and then injected i.v. in the caudal vein with either USPIO-P1, USPIO-P7 or USPIO-NSP at a dose of 100 µmol Fe/kg b.wt. (about 9 nmol of peptide/kg b.wt.) for MRI studies. Images were then acquired on a Bruker Biospec imaging system equipped with a Pharmascan bore (Ettlingen, Germany) working at 7T with a T₂-weighted Rapid Acquisition with Relaxation Enhancement (RARE) imaging protocol (TR/TE = 4000/80 ms, RARE factor = 4, NEX = 4, matrix = 256 x 256 µm, FOV = 3.5 x 2.5 cm, slice thickness = 1.1 mm, spatial resolution = 137 x 97 µm, TA = 17 min 4 sec). A small-animal monitoring and gating system was used to monitor animal respiration rate. During the acquisition of MR images, the mouse body temperature was maintained at 36-37°C using a warm water circulating system.

### Results

### 1. Affinity of gal-1-targeted phage clones and analysis of the expressed peptides

Fifty clones were arbitrarily isolated from the last rounds of each PDE. Among them, 16 phage clones were selected from PDE1 and 24 from PDE2 based on their first screening on gal-1 and control proteins (HSA and collagen). After deciphering the amino acid sequence of their expressed peptides, 8 different peptides were identified, several clones expressed the same peptide (Table 2). The most notable is P1, which is expressed by 30 clones (75% of the total number of clones) issued both from PDE1 (15 clones) and PDE2 (15 clones). The probability (P) of this peptide to be expressed by at least one clone within the phage display library is relatively low (about 21%), which means that its selection was not favored by its frequency but most likely by its gal-1 specificity.

**Table 2 Amino acid sequence of the selected peptides and the probability (P) of their expression by "k" phage clones in the library (P(k>0)). The consensus amino acid motifs are underlined**

| **Peptide** | **Number of expressing clones** | **Amino acid sequence** | **P (k>0)** |
|---|---|---|---|
| **P1/PD1** | 30 (15 from PD1 and 15 from PD2) | HLWGWLYAPSFQ | 20.71 |
| **P2/PD1** | 1 | YTFHFDIFQPHF | 0.18 |
| **P3/PD2** | 2 | HYSWSWIAYSPG | 47.20 |
| **P4/PD2** | 2 | VHWDFRQWWQPS | 0.40 |
| **P5/PD2** | 1 | YHHSGPYAGPMW | 62.10 |
| **P6/PD2** | 1 | SVYVEISWVRTM | 4.10 |
| **P7/PD2** | 2 | YSWHIDIVAPRN | 12.60 |
| **P8/PD2** | 1 | DWSSWVYRDPQT | 38.20 |

The amino acid frequency in the sequences of 8 identified peptides has been analyzed and represented in Figure 1A and in the sequence listing below. The most frequent amino acids are Leu, Ser and Trp (frequency superior to the mean ± SD), whereas Ala, Gly, Pro, Gln, His, Phe and Tyr have frequencies that surpass the mean and most of them are hydrophobic. Most of these amino acids are distributed in consensus motifs that can be identified in each of the 8 identified peptides (Table 2). Being an aliphatic nonpolar amino acid, Leu has a lateral group often found within a-helices of proteins; it can interact with similar residues by hydrophobic interactions. Ser is a polar amino acid that can contract hydrogen bonds with other residues via its -OH group. Trp is an aromatic non-polar amino acid bearing an indole side chain that can interact with other aromatic side chains by non-covalent stacking. Gly confers a high flexibility to the polypeptide backbone, while Pro can act as a hydrogen bond acceptor and contributes to the formation of β turns.

In the case of human gal-1, Trp68 and His52 are essential for the correct alignment of carbohydrate ligands at the CRD through a stacking interaction. Trp, Phe and Tyr residues present in our peptides could interpose between planar side chains of gal-1 amino acid residues and contract stacking interactions. Moreover, the retrieved peptides are rich in basic and polar residues that could establish hydrogen bonds with His44, His52, Arg48, Arg73, Asn46 et Asp54 of gal-1, which are currently interacting with natural carbohydrate ligands.

The affinity constants (Ka = 1/K*_{d}) against gal-1 or control proteins (HSA and collagen) were determined for one representative clone per peptide sequence. The specific affinity has been calculated by relating the Ka for gal-1 to the mean of Ka values for control proteins (Table 3). Based on this parameter, the best peptide clones were found to be P2 and P7. However, P1 presents a particular interest because of its high frequency of selection even when challenged with the stringent conditions of PDE2. The synthesized peptides P1 and P7 (Figure 1B) were selected for subsequent characterization. Their K*_{d} values against gal-1 are of 2.94·10⁻⁵ M for P1 and of 1.26·10⁻⁵ M for P7 (Figure 1C).

**Table 3 Affinity (expressed by Kₐ = 1/K*_{d}) ratios of the 8 peptide sequences expressed by the selected phage clones issued from the two phage display experiments. To note: The phage clone has a better specific affinity when the affinity ratio is high. Coll = collagen**

| **Selected peptides** | **Affinity 1 Kₐ^{Gal-1} / Kₐ^{HSA}** | **Affinity 2 Kₐ^{Gal-1} / Kₐ^{Coll}** | **Affinity 3 Kₐ^{Gal-1}/ ((Kₐ^{HSA} + Kₐ^{Coll})/2)** |
|---|---|---|---|
| **P1/PDE1 and PDE2** | 1.19 | 0.80 | 0.95 |
| **P2/PDE1** | 121.56 | 8.15 | 25.66 |
| **P3/PDE2** | 0.99 | 1.35 | 1.14 |
| **P4/PDE2** | 1.18 | 1.93 | 1.47 |
| **P5/PDE2** | 1.25 | 1.58·10¹³ | 2.50 |
| **P6/PDE2** | 1.17 | 1.17 | 1.17 |
| **P7/PDE2** | infinite ratio | infinite ratio | infinite ratio |
| **P8/PDE2** | 1.23 | 27.92 | 2.36 |

A BLAST (Basic Local Alignment Search Tool) search has been performed in order to check sequence similarities of peptides P1 and P7 with well-known proteins potentially involved in gal-1-regulated biological phenomena, such as embryonic development of the nervous system, angiogenesis, apoptosis, immunity, cell-cell and cell-matrix interaction, etc. The different sequence homologies of both peptides are listed in Table 4. The interest of peptides is thus increased if their sequences have homologies with proteins having high probability of interaction with gal-1. These proteins generally have carbohydrate moieties recognized by the CRD of gal-1.

Homology has been found between the amino acid sequence of P1 and that of the parathyroid hormone 2 receptor. This membrane receptor contains N-glycosylation sites which can thus be recognized by gal-1. Sequence similarities have also been found between P1 and neurogenin-3, which is a transcription factor playing a role in neurogenesis. Gal-1 has been found to be involved in nerve structure development and in the pathologies of the nervous system. The Ephrin type-A receptor 8 is implied in the regulation of biological processes during embryonic development of the nervous system. Gal-1 has been reported overexpressed in embryonic tissues, where it is involved in neuronal development and angiogenesis. A well-known process in which gal-1 is implied is apoptosis. It appears that P1 has sequence homologies with the apoptosis regulator Bcl-2 and with Fas apoptotic inhibitory molecule 3. This last one is involved in diverse phenomena like cell defense response, immune system and negative regulation of apoptosis.

P7 presents sequence similarities with membrane glycoproteins or proteins located in the extracellular matrix (mucin-16, ADAM-TS 10, semaphorin-6B, neuronal cadherin, tenascin) able to interact with gal-1. Indeed, as already mentioned above, gal-1 recognizes glycosylated moieties with its CRD and is also found in the extracellular matrix and at the membrane level. As an example, mucin-16 binds gal-1 with high affinity and seems to be implied in its secretion. P7 presents a high sequence homology with Neuronal Cadherin-2, a calcium-dependent glycoprotein of cell adhesion that acts during the nervous system development. Semaphorin-6B having also similarities plays a role in the axonal growth cone guidance. Gal-1 has been reported in high amount in the nervous system, where it contributes to the sensorial nerve development, the peripheral axon regeneration and the adult neurogenesis. Finally, the Death-inducer obliterator 1 is implied in apoptosis as gal-1.

**Table 4 Sequence alignment of peptides 1 and 7 with relevant protein sequences (common amino acids are in bold). Both sequences were targeted against the UniProtKB/Swiss-Prot database. The sequence alignments were performed using BLAST as implemented on the UniProt website, according to an automatic selection of the similarity matrix, and an E-value threshold of 10. Gaps were allowed without any filtering option**

| **Protein name** | **SwissProt ID** | **Peptide alignment** |
|---|---|---|
| **Peptide 1 - HLWGWLYAPSFQ** | | |
| Parathyroid hormone 2 receptor | P49190.1 | ¹ **H**L**WGWL** ⁶ |
| | | ⁹ **H**V**WGWL** ¹⁴ |
| Neurogenin-3 | Q9Y4Z2.2 | ³ **WG**W**LY**A**P** ⁹ |
| | | ¹⁶⁹ **WG**S**LY**S**P** ¹⁷⁵ |
| L-selectin | P14151.2 | ² **LWGW** ⁵ |
| | | ²⁰ **LWGW** ²³ |
| | | ³ **W**G**W** ⁵ |
| | | ⁹⁸ **W**T**W** ¹⁰⁰ |
| DnaJ homolog subfamily C member 14 | Q6Y2X3.2 | ³ **WGWL** ⁶ |
| | | ³⁹⁶ **WGWL** ³⁹⁹ |
| Semaphorin-4F | 095754.2 | ⁵ **WL**Y**AP**S**F** ¹¹ |
| | | ²²¹ **WL**N**AP**A**F** ²²⁷ |
| Semaphorin-4D | Q92854.1 | ⁵ **WL**YA**PSF** ¹¹ |
| | | ²⁰⁵ **WL**NE**PSF** ²¹¹ |
| Ephrin type-A receptor 8 | P29322.2 | ³ **WGWL** ⁶ |
| | | ⁴⁴ **WGWL** ⁴⁷ |
| Apoptosis regulator Bcl-2 | P10415.2 | ¹ **HL-**-**W-----GW-----LY**A**PS** ¹⁰ |
| | | ¹⁸⁴ **HL**HT**W**IQDNG**GW**DAFVE**LY**G**PS** ²⁰⁵ |
| Neurexophilin-1 | P58417.1 | ² **LW**G**WL** ⁶ |
| | | ⁸⁷ **LW**D**WL** ⁹¹ |
| Fas apoptotic inhibitory molecule 3 | 060667.1 | ⁵ **WL**Y**APS** ¹⁰ |
| | | ³⁵⁴ **WL**H**APS** ³⁵⁹ |
| NKG2-D type II integral membrane protein | P26718.1 | ⁶ **LYA**P**SF** ¹¹ |
| | | ¹⁹¹ **LYA**S**SF** ¹⁹⁶ |
| Brain-specific angiogenesis inhibitor 1-associated protein 2-like protein 1 | Q9UHR4.2 | ⁴ **GWLY** ⁷ |
| | | ³⁷⁷ **GWLY** ³⁸⁰ |
| **Peptide 7 - YSWHIDIVAPRN** | | |
| Ovarian cancer-related tumor marker CA125 ; Mucin-16 | Q8WXI7.2 | ⁷ **IVAPRN** ¹² |
| | | ²⁷³² **IVAPRN** ²⁷³⁷ |
| A disintegrin and metalloproteinase with thrombospondin motifs ; ADAM-TS 10 | Q9H324.2 | ¹ **YSWH** ⁴ |
| | | ⁸²⁷ **YSWH** ⁸³⁰ |
| | | |
| | | ³ **W**HID**IV** ⁸ |
| | | ³¹⁷ **W**QKS**IV** ³²² |
| Semaphorin-6B | Q9H3T3.4 | ⁸ **VAPR**N ¹² |
| | | ³⁶ **VAPR**D ⁴⁰ |
| Cadherin-2 ; Neural cadherin | P19022.4 | ⁵ **IDIV** ⁸ |
| | | ²⁵¹ **IDIV** ²⁵⁴ |
| | | |
| | | ⁶ D**IV**A**PR** ¹¹ |
| | | ¹⁴⁰ E**IV**F**PR** ¹⁴⁵ |
| Tenascin ; Glioma-associated-extracellular matrix antigen | P24821.3 | ⁹ **APRN** ¹² |
| | | ⁸⁹⁵ **APRN** ⁸⁹⁸ |
| Death-inducer obliterator 1 | Q9BTC0.5 | ⁹ **APRN** ¹² |
| | | ⁵⁶² **APRN** ⁵⁶⁵ |

### 2. Theoretical biochemical parameters of candidate peptides

The theoretical biochemical parameters of P1 and P7 are represented in Table 5. The peptide half-life was estimated according to the N-end rule and the probability of ubiquitin-degradation based on the presence of positively charged destabilizing residues, such as Lys, at the N-terminus of a polypeptide backbone. Both peptides have comparable half-life times, with a slightly longer value for P1 (3.5 hours) as compared to P7 (2.8 hours).

Aliphatic index is defined as the volume occupied by the aliphatic side chains. The high values for both P1 and P7 indicate a hydrophobic character, which was confirmed with the GRAVY (Grand Average of Hydropathy) parameter that is given by the ratio between the sum of all amino acid hydropathy values and the number of residues in the peptide sequence. However, P7 is more hydrophilic compared to P1, as suggested by LogP, LogD and the percentage of acid, basic and polar residues in its composition (50.1% vs 33.4% for P1).

**Table 5 Theoretical biochemical parameters of P1 and P7 calculated by using the ExPASy proteomics server, proteomics and sequence analysis tools. LogP and LogD were calculated by using the MarvinSketch 6.3.0 software (2014, http://www.chemaxon.com). Half-life was theoretically estimated in mammalian reticulocytes in vitro. LogP: partition coefficient, LogD: distribution coefficient estimated at pH 7.4 and a salt concentration of 150 mM.**

| **Parameter** | **Peptide 1** | **Peptide 7** |
|---|---|---|
| **pI** | 6.74 | 6.74 |
| **Estimated half-life** | 3.5 hours | 2.8 hours |
| **Aliphatic index** | 73.33 | 97.50 |
| **GRAVY** | -0.033 | -0.358 |
| **LogP** | 4.17 ± 1.02 | 1.02 ± 1.02 |
| **LogD_{7.4}** | -2.1 | -7.63 |
| **Residues (%)** | | |
| **Acid** | 0 | 8.3 |
| **Basic** | 8.3 | 16.6 |
| **Nonpolar** | 66.6 | 49.9 |
| **Polar** | 24.9 | 24.9 |

### 3. Analysis of spatial conformation of peptides 1 and 7

The secondary structure and aggregation properties of our peptides in solution (distilled water) were studied by circular dichroism. The spectra of peptides were compared with the spectroscopic signatures derived from proteins with well-known secondary structures, such as the myoglobin which is mainly composed of a-helices, lysozyme of β-turns, β-lactoglobulin of β-sheets, and ferredoxin that has a random coil state. According to the spectra (data not shown), the CD signatures of P1 and P7 are similar to that of ferredoxin, indicating that they adopt a random coil state in aqueous solution. This signifies that, despite their hydrophobic amino acids, the peptides do not have tendency to aggregate in β sheets. No difference was observed in the behavior of peptides when temperature is modified (data not shown), which stressed that the random coil state is predominant for the conformation of these peptides. Computational sequence analyses have also confirmed the absence of secondary structure elements for both peptides and their flexibility in solution by employing domain scanning (InterPro5), motif scanning (Motif Scan) and protein disorder analyses (disEMBL 1.5). This characteristic suggests their ability to interact with the target, gal-1. Frequently, short sequence peptides adopt a plethora of random coil conformations due to their high dynamics.

### 4. Docking study of the interaction of peptides with gal-1

The best consensus models related to P1 and P7 binding take place in the CRD of gal-1. However, they have a different binding mode in a conserved region, meaning that P7 in its extended conformational state has a perpendicular position compared to P1 orientation. P1 conformational state is curled up within the CRD core (Figure 2). As a consequence, the residues of gal-1 able to interact with peptides, i.e., located at a distance below 4 Å, are more numerous for P7 in light of its extended conformation (Figure 3). Nevertheless, twelve gal-1 residues (2 acidic, 3 basic, 4 uncharged polar and 3 hydrophobic) are identical (underlined in bold), which reveals that a similar interaction frame appears (Figure 3, right). Interestingly, numerous gal-1 residues in contact with P1 or P7 are similar with those implied in the interaction with the natural sugar ligand (Figure 3), which indicates the potential efficiency of P1/P7 peptides as gal-1 ligands. To highlight the interactions at play in both complexes, we inspected the gal-1 residues that surround each peptides' residue. Several peptide residues are close to gal-1 residues involved in carbohydrate binding mode. For instance, Trp68, a key residue for the optimum alignment of carbohydrate ligands at the CRD, is close to Tyr7 (P1) and His4 (P7), both of them being able to interact with aromatic residues by stacking interactions. His44, a polar residue involved in H-bonding of carbohydrates, is located in the vicinity of Tyr7 and Phe11 for P1, suggesting plausible stacking interactions, but closed to Asp6 in the case of P7, a closeness that opens up the possibility for hydrogen bonds interactions. Similarly, other gal-1 residues such as Arg48, Asn61 and Glu71, which are known to play a role in carbohydrate binding via H-bonds interactions, are all close to aromatic residues, notably Tyr7 (P1) and Trp3 (P7), both being involved in hydrogen bonding. Although our complexes are consensus solutions issued from docking calculations, such proximity of residues related to carbohydrate binding consolidates the hypothesis that both P1 and P7 could adopt interactions that are similar to the ones of the carbohydrate-binding mode. Let us note that the Root Mean Square Deviation (RMSD) value between gal-1 interacting with P1 (A) and the one with P7 (B) is weak (∼0.47 Å), which emphasizes that there are no important conformational changes upon P1/P7 binding to gal-1.

### 5. Validation of P1 and P7 binding to thyroid cancer biopsies

The ability of P1 and P7 to target gal-1 expressed by human tumor tissues has been evaluated by immunohistochemistry on human papillary thyroid cancer biopsy sections (Figure 4). As detected by anti-gal-1 antibody, all papillary thyroid cancer cases showed a particularly high expression of gal-1 in the tumor areas, but also in the stroma, with a cytoplasmic, nuclear and extracellular location. In the extracellular space, gal-1 contributes to the cancer cell adhesion to the extracellular matrix. P1 and P7 (but not the negative control peptide, NCP) presented an efficient targeting of the papillary areas, but not of the stroma or the healthy structures, their cellular location corresponding to the one of gal-1 (Figure 4).

Gal-1 was also overexpressed in the 4 studied cases of follicular thyroid cancer (data not shown), another well-differentiated thyroid cancer; the staining was observed overall in the cytoplasm and the nucleus, but also sometimes in the colloid of the thyroid follicle. Similar pattern of gal-1 expression was observed in the 6 cases of adenomas (data not shown), benign lesions of thyroid, and the 6 cases of anaplastic thyroid cancer (data not shown), a non-differentiated lesion. We have noticed a staining in the 5 cases of healthy (data not shown) and 2 cases of inflammatory (data not shown) thyroid. Gal-1 is mainly located in the cytoplasm and the nucleus of the follicular cells.

The gal-1 staining in healthy thyroid and in various pathological conditions was analyzed on immunohistochemistry microphotographs using the ImageJ software and the results are presented in Figure 4. Gal-1 expression is higher in malignant cases compared to healthy and inflammatory thyroid. Moreover, the more aggressive anaplastic thyroid cancer is characterized by the highest gal-1 expression. However, the papillary and follicular thyroid cancers present a similar level of gal-1 expression, which is inferior to that of anaplastic thyroid cancer, but higher than in adenoma or healthy and inflammatory thyroid. Gal-1 staining produced by P1 and P7 is weaker in healthy thyroids and thyroid adenomas when compared to papillary, follicular and anaplastic thyroid cancer.

### 6. Co-localization of P1 and P7 with gal-1 expressed by TPC-1 cells

In order to evaluate the specific binding of P1 and P7 to gal-1, they were co-localized with the target on TPC-1 cells, which express gal-1 in their cytoplasm (Figure 5).

### 7. Influence of P1 and P7 on the adhesion of TPC-1 to gal-1

Gal-1 has been reported to interact with tumor cells and to facilitate their adhesion to the extracellular matrix, leading to metastasis. Since P1 and P7 bind to gal-1, we would expect that TPC-1 cell adhesion to gal-1 might be prevented in their presence. Aiming to verify this hypothesis, the effect of peptides on TPC-1 cell adhesion to gal-1 has been assessed (Figure 6A). Both P1 and P7 induced a similar significant (p<0.05) decrease of cell adhesion (around 25% and 30%, respectively) at 500 nM. P1Scr did not induce any significant effect on TPC-1 cell adhesion, representing a good negative control.

### 8. Detection of caspase-3 in TPC-1 cells after treatment with peptides

Since both peptides prevented the adhesion of TPC-1 cells to gal-1, we verified the hypothesis that the decreased number of adhered cells is indeed produced by peptide binding to gal-1 and not by the cell death induced by apoptosis. Apoptotic cells were observed by immunofluorescence via the detection of active caspase-3 (Figure 6B). As compared with positive control cells treated with 2µM camptothecin, where caspase-3 was active, peptides did not produce any significant apoptotic effect on TPC-1 cells.

### 9. In vitro evaluation of USPIO derivatives

The selected peptides were grafted to USPIO and their ability to bind to gal-1 was first validated by ELISA, used to determine the K*_{d} of USPIO-P1 and USPIO-P7 (Figure 7A). The K*_{d} values were of 2.05·10⁻⁷ M (for USPIO-P1) and 7.56·10⁻⁸ M (for USPIO-P7) respectively, proving thus that both peptides conserved their ability to recognize gal-1 after covalent coupling to USPIO. They were also able to specifically bind to gal-1 expressed by TPC-1 cells, as revealed by Perls'-DAB staining (Figure 7B) and USPIO co-localization with gal-1 by immunofluorescence (Figure 7C). Used as a negative control, USPIO functionalized with a non-specific peptide (USPIO-NSP) does not show a specific affinity towards gal-1 and slightly binds to cancer cells, corroborating thus the specific gal-1 binding of USPIO-P1 and USPIO-P7.

Similar experiments were performed with other tracers and radio-labels with similar results (data not shown).

### 10. Evaluation of the competition between USPIO derivatives and anti-gal-1 antibody

Aiming to verify the specific binding of USPIO derivatives to gal-1 expressed by TPC-1 cells, a competition experiment has been performed between anti-gal-1 antibody and the functionalized contrast agents. The binding of anti-gal-1 antibody to TPC-1 cells is significantly decreased when incubated simultaneously with USPIO-P1 or USPIO-P7 (p<0.05). This decrease is more important with USPIO-P7 (Figure 8). USPIO-P1 and USPIO-P7 compete with the same target as the anti-gal-1 antibody but not with the same efficiency. This result is in agreement with K*_{d} values of USPIO derivatives, which indicate a higher affinity towards gal-1 for USPIO-P7.

### 11. Cytotoxic evaluation of peptides and corresponding USPIO derivatives

The cytotoxic effects of peptides and of their USPIO derivatives were evaluated by a MTT assay on TPC-1 and HepaRG cell lines (data not shown). The non-biotinylated peptides (P1 and P7) and their USPIO derivatives (USPIO-P1 and USPIO-P7) were assessed at various concentrations and times of incubation (2h, 24h).

The results prove that P1 (p<0.05) and P7 (p<0.01) induce a significant effect on cell viability of about 10-13% when incubated at 500 nM during 24 hours. Knowing that peptides do not induce apoptosis in the same experimental conditions, we assume that inhibition of cell adhesion might be responsible of the apparent decrease of cell viability as documented by the experiments presented above. Higher concentrations of peptides did not produce a significant decrease of cell viability, probably because of a competition between peptide molecules at the targeted site by steric hindrance. No significant lethal effects were observed in any of the other experimental conditions either for peptides, USPIO derivatives or cell types.

### 13. In vivo imaging

Figure 9 shows MR images of TPC-1 tumors xenografted in athymic nude male mice before (pre-contrast) and after (post-contrast) the injection of either USPIO-P1, USPIO-P7 or USPIO-NSP. USPIO-NSP is a negative control contrast agent functionalized with a non-specific peptide (NSP).

The results show that both USPIO-P1 and USPIO-P7 (but not USPIO-NSP) induce a strong negative contrast of TPC-1 tumors on MR images.

### Discussion

Papillary carcinoma is the most frequent well-differentiated thyroid cancer, accounting for about 80% of all thyroid cancers. The increasing incidence is mainly due to overdiagnosis of thyroid nodules, which are routinely detected by ultrasound imaging. Diagnosis of papillary thyroid carcinoma generally relies on the FNAC followed by histological observation. However, this technique is inconclusive and about 90% of the thyroidectomies are performed for benign nodules. As the diagnosis will define the future treatment and management of patients, a diagnostic challenge emerged. It consists in the ability to discriminate the benign adenomas from well-differentiated thyroid cancers using a non-invasive diagnostic method and reduce in this way the number of useless and painful surgeries.

Several studies have already proved that gal-1 is a good biomarker of cancers. Indeed, this protein is overexpressed in a large variety of cancers, whom well-differentiated thyroid cancers and plays key roles in tumor biology. Among other lectins, galectins constitute a conserved family of proteins (fifteen mammalian members), which shares the CRD, a consensus sequence of about 130 amino acids allowing the binding to N-and O-linked glycans.

The expression of gal-1 between healthy and different pathological thyroid cases was evaluated. Based on the studied cases, this protein is always significantly overexpressed in thyroid cancers (papillary, follicular or anaplastic) compared to healthy or inflammatory thyroid, corroborating the current literature. Both gal-1 and gal-3 have been proposed as markers of thyroid malignancy because of their ability to distinguish benign nodules and well-differentiated thyroid cancers, based on their overexpression in tumors and in metastatic tissues. Moreover, gal-1 has been proposed as a target to inhibit papillary thyroid carcinoma growth and metastasis.

In this study, gal-1 expression has been reported in thyroid adenomas, although at a lower level than in malignant cases. Characterized by a microfollicular architecture, thyroid adenomas are considered as benign encapsulated tumors and are common in adult population. Sometimes, if certain mutations (N-RAS and K-RAS) are present, follicular adenomas can evolve in follicular carcinoma. This feature could explain the lectin expression observed in the studied cases.

Concerning the expression of gal-1 in inflammatory thyroid cases, no significant difference was observed with the healthy cases. A higher expression of gal-1 in anaplastic carcinoma was also noted compared to follicular carcinoma and thyroid adenoma. Anaplastic cancer is an undifferentiated thyroid cancer with the worst prognosis, the median survival being of 9 weeks and a 5-year survival of 7%. The overexpression of gal-1 in tumors is considered as an indicator of tumor development and consequently a sign of severe prognosis. The explanation would be the implication of this lectin in the tumor immune-escape, angiogenesis and metastasis phenomena.

In the present work, the phage display technique has been employed to identify random sequence 12-mer linear peptides specifically targeted to gal-1. Eight peptides were identified which were able to bind to gal-1 and which are suitable for use in the visualization of the thyroid malignancies and for therapeutic use. Two 12-mer peptides, P1 (SEQID n° 1) and P7 (SEQ ID n° 7), were evaluated further. They were selected according to their specific affinity towards gal-1 and their co-localization with the target in thyroid tumor cells (TPC-1). P1 and P7 (but not the negative control peptide, NCP) have both the ability to bind to pathological thyroid tissues, recognizing in particular the papillary areas of papillary carcinomas. Moreover, the binding of both peptides is higher in malignant thyroid tissues compared to healthy thyroid or benign lesions. This property is an advantage for *in vivo* molecular imaging, allowing the discrimination of this type of cancer with a lower background.

The circular dichroism studies indicated that P1 and P7 have a random coil conformation and do not show signs of β-sheet aggregates. Our docking study assumes that both P1 and P7 locate in a conserved area of the gal-1 CRD with a different spatial disposition. In terms of possible binding modes, P1 (more flexible than P7) is curled up whereas P7 has a perpendicular position in the CRD. The latter peptide can interact with a larger number of amino acids in the CRD. Moreover, several gal-1 residues in contact with P1 or P7 are similar to those in interaction with the natural carbohydrate ligand.

A thorough in silico study on peptides 1 and 7 confirmed the probability of interaction with gal-1, first based on sequence homologies with glycoproteins or matrix proteins. Indeed, gal-1 has the property of binding to glycosylated residues via its CRD. Globally, the proteins presenting a sequence identity with our peptides are implied in neuronal development, apoptosis or immunosurveillance. All of these processes have been reported to involve gal-1, corroborating the high probability of interaction with P1 and P7. They could interact with the CRD through stacking and hydrogen-bonding interactions engaged in the recognition of carbohydrates as our docking simulations have reported above.

These interactions in the CRD could interfere with gal-1 functions. In fact, P1 and P7 (but not P1Scr) induce a significant decrease of TPC-1 cell adhesion, which ranges between 25% and 30% when incubated at 500 nM. Since caspase-3 is not activated by these peptides, the decreased cell number observed by both adhesion and MTT assays is explained by peptide binding to the target, preventing thus the cell adhesion to gal-1. The specific binding of P1 and P7 to gal-1 is additionally confirmed by the absence of any effect on cell adhesion when cells are incubated with P1Scr. It is well known nowadays that gal-1, among other secreted galectins, interacts with integrins and extracellular matrix glycoproteins (i.e., laminin, fibronectin), regulating cell-cell and cell-matrix adhesion and in this way the cell migration and tumor progression. Gal-1 mediates the smooth muscle cell adhesion by interaction with β1 integrin, contributing to atherosclerosis and restenosis, and promotes the migration, invasion and epithelial-mesenchymal transition of lung cancer cells via its binding to α6β4 integrin. It has been demonstrated that tumor cell adhesion could be inhibited using short peptides targeting gal-3 CRD. In this study, peptides antagonist of gal-3 CRD have been isolated by phage display and showed effects on breast carcinoma cells' adhesion and aggregation by blocking the interaction between the target and the Thomsen-Friedenreich glycoantigen. Due to their interaction with gal-1, peptides P1 and P7 can prevent tumor invasion, but also the intracellular signaling pathways regulated by integrin activation. As a consequence, the peptides according to the embodiments of the current invention can be used for therapeutic purpose as well.

Altogether, these results confirm the efficiency of P1 and P7 to interact with gal-1. Once grafted to a contrast agent for MRI, these peptides were shown to be able to assist the diagnosis of papillary thyroid cancer by specific binding to gal-1 and accumulation in the tumor areas. A reason to focus on papillary thyroid cancer is that the incidence is high and the prognostic remains good, whereas anaplastic cancer is rare but the most aggressive, its survival rate being of about 2-5 %. Thyroid nodules are generally diagnosed with ultrasound during unrelated pathology examinations. Radionuclide imaging, with high sensitivity, is limited by low spatial resolution, while exposition to ionizing radiation can be harmful for the patients at long term. Instead, MRI offers numerous advantages in the field of cancer imaging and staging of the tumor development, such as a better soft tissue contrast, an excellent spatial resolution and non-invasiveness. Nevertheless, its sensitivity is low considering that contrast agents must be injected in micromolar doses to observe significant signal changes. With the help of vectorized contrast agents (gadolinium chelates or iron oxide nanoparticles), MRI becomes then a real sensitive and specific imaging technique.

It will be understood that other imaging techniques and suitable tracers or contrast agents are equally within the scope of the current invention as they are equally suitable to visualize the thyroid carcinomas.

Before and after the covalent coupling to USPIO, the binding of peptides P1 and P7 to gal-1 expressed by a papillary thyroid cancer cell line, TPC-1, has been confirmed by iron staining and by immunofluorescence, which allowed us to observe the co-localization of our functionalized contrast agents with their target. The specific recognition of gal-1 by P1 and P7 was corroborated by the weak binding of USPIO functionalized with a non-specific peptide. It has also been proved that USPIO-P1 and USPIO-P7 are gal-1 specific as they compete with anti-gal-1 antibody for gal-1 binding in cancer cells. The absence of cytotoxicity in a human hepatocyte cell line after 2 or 24 hours of incubation with peptides or with USPIO derivatives is positive and promising for future clinical applications.

In summary, this study shows that by targeting of gal-1 with short synthetic peptides fused to a tracer or contrast agent, a targeting agent and aid for the diagnosis for thyroid cancer is presented. Moreover, as a large variety of human cancers overexpress this protein, our vectorized nanoparticles could be also employed for their imaging diagnosis in other cancer types, organs or tissues.

Secondly, the synthetic peptides according to the current invention may equally be used as therapeutic agents in thyroid cancer therapy, e.g. by preventing tumor adhesion and/or influencing the intracellular signaling pathways regulated by integrin activation.

It is supposed that the present invention is not restricted to any form of realization described previously and that some modifications can be added to the presented example of fabrication without reappraisal of the appended claims.

### Amino acid sequence of peptides 1 to 8

P1 - SEQ ID n°1: HLWGWLYAPSFQ
P2 - SEQ ID n°2: YTFHFDIFQPHF
P3 - SEQ ID n°3: HYSWSWIAYSPG
P4 - SEQ ID n°4: VHWDFRQWWQPS
P5 - SEQ ID n°5: YHHSGPYAGPMW
P6 - SEQ ID n°6: SVYVEISWVRTM
P7 - SEQ ID n°7: YSWHIDIVAPRN
P8 - SEQ ID n°8: DWSSWVYRDPQT

## Claims

1. Peptide capable of binding to galectin-1, **characterized in that** said peptide has at least 95% sequence identity to an amino acid sequence chosen from SEQ:ID n° 1 to8.

2. Peptide according to claim 1, **characterized in that** said peptide has at least 99% sequence identity with an amino acid sequence chosen from SEQ:ID n° 1 to 8.

3. Peptide according to any of the previous claims, **characterized in that** said peptide has an amino acid sequence chosen from SEQ:ID n° 1 to 8.

4. Peptide according to any of the previous claims, **characterized in that** said peptide has an amino acid sequence chosen from SEQ:ID n° 1, 2 or SEQ:ID n°7.

5. Peptide according to any of the previous claims, **characterized in that** said peptide is linked to a tracer or contrast agent suitable for being used in a medical imaging methodology.

6. Peptide according to claim 5, **characterized in that** said tracer or contrast agent is useful for MRI and chosen from superparamagnetic iron oxide (SPIO), ultrasmall superparamagnetic iron oxide (USPIO), paramagnetic ion based contrast agent such as a gadolinium based contrast agent, a manganese based contrast agent, dysprosium based contrast agent, europium based contrast agent or a holmium based contrast agent.

7. Peptide according to claim 5, **characterized in that** said tracer is useful for Computed Tomography (CT) such as X-Ray, bimodal PET-MRI, PET, PET-CT or SPECT and is chosen from short half-life isotopes such as carbon-11 (¹¹C), nitrogen-13 (¹³N), oxygen-15 (¹⁵O), fluorine-18 (¹⁸F), gallium-68 (⁶⁸Ga), zirconium-89 (⁸⁹Zr), or rubidium-82 (⁸²Rb), copper-64 (⁶⁴Cu), copper-62 (⁶²Cu), copper-60 (⁶⁰Cu), iodine-124 (¹²⁴I), brom-76 (⁷⁶Br), indium-111 (¹¹¹In), iodine-123 (¹²³I), iodine-124 (¹²⁴I), iodine-125 (¹²⁵I), iodine-131 (¹³¹I), tellurium-125m (^{125m}Te), thulium-170 (¹⁷⁰Tm), lutetium-177 (¹⁷⁷Lu), Rhenium-186 (¹⁸⁶Re), Rhenium-188 (¹⁸⁸Re), astatine-211 (²¹¹At), galliun-67 (⁶⁷Ga), galliun-66 (⁶⁶Ga), galliun-68 (⁶⁸Ga), technetium-99m (^{99m}Tc), yttrium-86 (⁸⁶Y), zirconium-89 (⁸⁹Zr), cobalt-55 (⁵⁵Co), strontium-83, (⁸³Sr), manganese-51 (⁵¹Mn), manganese-52 (⁵²Mn), fer-52 (⁵²Fe), or contrast generating elements such as iodine, gold, bismuth, bromine, tantalum, platinum, ytterbium, yttrium, gadolinium, tungsten.

8. A diagnostic or therapeutic composition for cancer or malignancies of the thyroid gland, comprising one or more peptides according to claims 1 to 7.

9. Method for imaging galectin-1 expressing tissues and/or organs in a subject, **characterized in that** a galectin-1 binding peptide according to any one of the claims 1 to 7 is administered to said subject.

10. Method according to claim 9, **characterized in that** said imaging method is MRI, CT, PET, SPECT, PET-CT, X-Ray, bimodal PET-MRI, fluorescent imaging or multimodal molecular imaging.

11. Method according to any of the previous claims, **characterized in that** malignancies of the thyroid or thyroid nodules are visualized.

12. Method according to any of the claims 9 to 11, whereby said imaging method is MRI and the peptide is administered to said subject in a dose of between 10 nmol of peptide per kg body weight of said subject and 100 µmol of peptide per kg body weight of said subject.

13. Method according to any of the claims 9 to 11, whereby said imaging method is a nuclear imaging technique and the peptide is administered to said subject in a dose of 1 nmol of peptide per kg body weight of said subject and 1 µmol of peptide per kg body weight of said subject .

14. A method for providing information for thyroid cancer diagnosis or malignancies of the thyroid gland, comprising the steps of:
c) administering a composition including the galectin-1 binding peptide of any one of claims 1 to 7 to a patient suspected of having thyroid cancer; and
d) imaging the thyroid region by means of a medical imaging methodology, preferably MRI.

15. Method according to any of the previous claims, **characterized in** said thyroid cancer is papillary thyroid cancer, follicular thyroid cancer, or anaplastic thyroid cancer.

16. Peptide according to any of the claims 1 to 4, for therapeutic use, especially treatment of cancer or malignancies or the prevention or inhibition of cancer growth, cell proliferation and/or metastasis, whereby said cancer is thyroid cancer.

17. A molecule which is a peptide mimetic of the peptides described in any of the claims 1 to 7.


SEQUENCE LISTING

<110>  Université de Mons

<120>  GALECTIN-1 BINDING PEPTIDES AND USE THEREOF FOR DIAGNOSTIC AND
       THERAPEUTIC PURPOSE

<130>  UMON-002-EP

<160>  40

<170>  PatentIn version 3.5

<210>  1
<211>  12
<212>  PRT
<213>  Human

<400>  1

His Leu Trp Gly Trp Leu Tyr Ala Pro Ser Phe Gln
1               5                   10


<210>  2
<211>  12
<212>  PRT
<213>  Human

<400>  2

Tyr Thr Phe His Phe Asp Ile Phe Gln Pro His Phe
1               5                   10


<210>  3
<211>  12
<212>  PRT
<213>  Human

<400>  3

His Tyr Ser Trp Ser Trp Ile Ala Tyr Ser Pro Gly
1               5                   10


<210>  4
<211>  12
<212>  PRT
<213>  Human

<400>  4

Val His Trp Asp Phe Arg Gln Trp Trp Gln Pro Ser
1               5                   10


<210>  5
<211>  12
<212>  PRT
<213>  Human

<400>  5

Tyr His His Ser Gly Pro Tyr Ala Gly Pro Met Trp
1               5                   10

<210>  6
<211>  12
<212>  PRT
<213>  Human

<400>  6

Ser Val Tyr Val Glu Ile Ser Trp Val Arg Thr Met
1               5                   10

<210>  7
<211>  12
<212>  PRT
<213>  Human

<400>  7

Tyr Ser Trp His Ile Asp Ile Val Ala Pro Arg Asn
1               5                   10

<210>  8
<211>  12
<212>  PRT
<213>  Human

<400>  8

Asp Trp Ser Ser Trp Val Tyr Arg Asp Pro Gln Thr
1               5                   10

<210>  9
<211>  6
<212>  PRT
<213>  Human

<400>  9

His Leu Trp Gly Trp Leu
1               5

<210>  10
<211>  6
<212>  PRT
<213>  Human

<400>  10

His Val Trp Gly Trp Leu
1               5

<210>  11
<211>  7
<212>  PRT

<213>  Human

<400>  11

Trp Gly Trp Leu Tyr Ala Pro
1               5


<210>  12
<211>  7
<212>  PRT
<213>  Human

<400>  12

Trp Gly Ser Leu Tyr Ser Pro
1               5


<210>  13
<211>  4
<212>  PRT
<213>  Human

<400>  13

Leu Trp Gly Trp
1


<210>  14
<211>  3
<212>  PRT
<213>  Human

<400>  14

Trp Gly Trp
1


<210>  15
<211>  3
<212>  PRT
<213>  Human

<400>  15

Trp Thr Trp
1


<210>  16
<211>  4
<212>  PRT
<213>  Human

<400>  16

Trp Gly Trp Leu
1

<210>  17
<211>  7
<212>  PRT
<213>  Human

<400>  17

Trp Leu Tyr Ala Pro Ser Phe
1               5


<210>  18
<211>  7
<212>  PRT
<213>  Human

<400>  18

Trp Leu Asn Ala Pro Ala Phe
1               5


<210>  19
<211>  7
<212>  PRT
<213>  Human

<400>  19

Trp Leu Tyr Ala Pro Ser Phe
1               5


<210>  20
<211>  7
<212>  PRT
<213>  Human

<400>  20

Trp Leu Asn Glu Pro Ser Phe
1               5


<210>  21
<211>  10
<212>  PRT
<213>  Human

<400>  21

His Leu Trp Gly Trp Leu Tyr Ala Pro Ser
1               5                   10


<210>  22
<211>  22
<212>  PRT
<213>  Human

<400>  22

His Leu His Thr Trp Ile Gln Asp Asn Gly Gly Trp Asp Ala Phe Val

1               5                   10                  15

Glu Leu Tyr Gly Pro Ser
            20

<210>  23
<211>  5
<212>  PRT
<213>  Human

<400>  23

Leu Trp Gly Trp Leu
1               5

<210>  24
<211>  5
<212>  PRT
<213>  Human

<400>  24

Leu Trp Asp Trp Leu
1               5

<210>  25
<211>  6
<212>  PRT
<213>  Human

<400>  25

Trp Leu Tyr Ala Pro Ser
1               5

<210>  26
<211>  6
<212>  PRT
<213>  Human

<400>  26

Trp Leu His Ala Pro Ser
1               5

<210>  27
<211>  6
<212>  PRT
<213>  Human

<400>  27

Leu Tyr Ala Pro Ser Phe
1               5

<210>  28

<211> 6
<212> PRT
<213> Human

<400> 28

Leu Tyr Ala Ser Ser Phe
1               5


<210> 29
<211> 4
<212> PRT
<213> Human

<400> 29

Gly Trp Leu Tyr
1


<210> 30
<211> 6
<212> PRT
<213> Human

<400> 30

Ile Val Ala Pro Arg Asn
1               5


<210> 31
<211> 4
<212> PRT
<213> Human

<400> 31

Tyr Ser Trp His
1


<210> 32
<211> 6
<212> PRT
<213> Human

<400> 32

Trp His Ile Asp Ile Val
1               5


<210> 33
<211> 6
<212> PRT
<213> Human

<400> 33

Trp Gln Lys Ser Ile Val
1               5

<210>  34
<211>  5
<212>  PRT
<213>  Human

<400>  34

Val Ala Pro Arg Asn
1               5


<210>  35
<211>  5
<212>  PRT
<213>  Human

<400>  35

Val Ala Pro Arg Asp
1               5


<210>  36
<211>  4
<212>  PRT
<213>  Human

<400>  36

Ile Asp Ile Val
1


<210>  37
<211>  6
<212>  PRT
<213>  Human

<400>  37

Asp Ile Val Ala Pro Arg
1               5


<210>  38
<211>  6
<212>  PRT
<213>  Human

<400>  38

Glu Ile Val Phe Pro Arg
1               5


<210>  39
<211>  4
<212>  PRT
<213>  Human

<400>  39

Ala Pro Arg Asn
1

<210> 40
<211> 20
<212> DNA
<213> Human

<400> 40
ccctcatagt tagcgtaacg 20